**Europäisches Patentamt**

(19) **European Patent Office**

**Office européen des brevets**

(11) Publication number: **0 210 279 B1**

(12) # EUROPEAN PATENT SPECIFICATION

(45) Date of publication of patent specification :
14.08.91 Bulletin 91/33

(21) Application number : 86900852.4

(22) Date of filing : 24.01.86

(86) International application number :
PCT/JP86/00031

(87) International publication number :
WO 86/04351 31.07.86 Gazette 86/17

(51) Int. Cl.⁵ : **C12N 9/72, C12N 15/58,**
**C12N 1/21, C12P 21/02**

(54) STABILIZED HUMAN PROUROKINASE.

(30) Priority : 25.01.85 JP 11033/85
20.04.85 JP 83611/85
20.07.85 JP 159294/85

(43) Date of publication of application :
04.02.87 Bulletin 87/06

(45) Publication of the grant of the patent :
14.08.91 Bulletin 91/33

(84) Designated Contracting States :
BE CH DE FR GB IT LI NL SE

(56) References cited :
EP-A- 0 092 182
JP-A- 5 951 300
JP-A-56 158 799
JP-A-58 148 899
JP-A-60 180 519
BIOTECHNOLOGY, vol. 3, no. 10, October 1985,
pages 923-929, New York, US; W. HOLMES et al.:
"Cloning and expression of the gene for pro-
urokinase in Escherichia coli"

(73) Proprietor : SAGAMI CHEMICAL RESEARCH
CENTER
4-5, Marunouchi 1-chome
Chiyoda-ku Tokyo 100 (JP)
Proprietor : CENTRAL GLASS COMPANY, LIMITED
5253, Oaza Okiube Ube-shi
Yamaguchi 755 (JP)
Proprietor : HODOGAYA CHEMICAL COMPANY,
LIMITED
4-2, Toranomon 1-chome Minato-ku
Tokyo 105 (JP)
Proprietor : NIPPON SODA CO., LTD.
2-1, Ohtemachi 2-chome
Chiyoda-ku, Tokyo 100 (JP)

Proprietor : NISSAN CHEMICAL INDUSTRIES LTD.
3-7-1, Kanda Nishiki-cho
Chiyoda-ku Tokyo (JP)
Proprietor : Tosoh Corporation
4560, Oaza Tonda
Shinnanyo-shi Yamaguchi-ken (JP)

(72) Inventor : MIYAKE, Toshio
4-4-1, Nishionuma Sagamihara-shi
Kanagawa 229 (JP)
Inventor : HIBINO, Yasuo
4-4-1, Nishionuma Sagamihara-shi
Kanagawa 229 (JP)
Inventor : KOBAYASHI, Yoichi
4-4-1, Nishionuma Sagamihara-shi
Kanagawa 229 (JP)
Inventor : WATABE, Ken
1-3-505, Hijiriyama Konan-ku, Yokohama-shi
Kanagawa 233 (JP)
Inventor : OMORI, Muneki
2-5-9, Zoshigaya Toshima-ku
Tokyo 171 (JP)
Inventor : MIKI, Tetsuzo
2-1-A-908, Shinshinden Abiko-shi
Chiba 270-11 (JP)
Inventor : YOKOYAMA, Midori
Daiichi Tachibana-so 201 5-4-7, Sagamiono
Sagamihara-shi Kanagawa 228 (JP)
Inventor : MATSUMOTO, Reiko
2621, Asamizodai Sagamihara-shi
Kanagawa 228 (JP)
Inventor : WATANABE, Kazuo
1-9-1, Minamidai
Sagamihara-shi Kanagawa 228 (JP)
Inventor : NUMAO, Naganori
1-9-2, Minamidai
Sagamihara-shi Kanagawa 228 (JP)

(74) Representative : Patentanwälte Grünecker,
Kinkeldey, Stockmair & Partner
Maximilianstrasse 58
W-8000 München 22 (DE)

Note : Within nine months from the publication of the mention of the grant of the European patent, any person may
give notice to the European Patent Office of opposition to the European patent granted. Notice of opposition shall be
filed in a written reasoned statement. It shall not be deemed to have been filed until the opposition fee has been paid
(Art. 99(1) European patent convention).

## Description

TECHNICAL FIELD

This invention relates to DNA segments coding for a stabilized human prourokinase-like polypeptide, plasmids containing the DNA segments, Escherichia coli containing the plasmid, and a process for preparing a human prourokinase-like polypeptide using the Escherichia coli.

The stabilized human prourokinases of the present invention are stable to various proteolytic enzymes, permitting them to be prepared at high yields and high purity. Furthermore, when administered to humans and animals, a long lasting efficacy may be anticipated from this substance due to its nonsusceptibility to enzymatic hydrolysis in vivo.

On the other hand, when a gene system involving a DNA segment coding for the human prourokinase-like polypeptide of the present invention is used, the polypeptide can be expressed at a high efficiency, permitting the polypeptide to be prepared with an economic advantage.

BACKGROUND ART

Human urokinase is an enzyme found as a trace in human urine capable of activating inactive plasminogen into plasmin, and the plasmin thus formed is capable of dissolving fibrin. This urokinase consists of two polypeptide chains linked together by a disulfide bond. On the contrary, human prourokinase is a single chain polypeptide in which the aforesaid two polypeptide chains are joined together through an amide bond. Although this prourokinase itself does not possess the aforementioned activity, it can be converted to the preceding active urokinase by cutting one amide bond. The urokinase is also referred to as mature urokinase.

Because of the aforesaid action, human urokinase is used as a thrombolytic agent. The use of human urokinase for intravenous administration requires a highly refined product, but urokinase in general is said to be an unstable enzyme being susceptible to hydrolysis during the purification process. For example, the urokinase prepared from human urine consists of a high molecular weight urokinase having a molecular weight of about 54,000 and a low molecular weight urokinase having a molecular weight of about 33,000, and the low molecular weight urokinase is formed by cleaving the high molecular weight urokinase with protease. It has been suggested that this cleavage site is a peptide bond between the two lysine residues positioned l35th and l36th respectively from the serine at the N-terminus of the high molecular weight matured urokinase. Such cleavage is caused not only during the purification process of prourokinase from human urine but also during the purification process of prourokinase produced by the recombinant DNA technology. Moreover, a similar cleavage is expected to be caused by a trypsin-like protease contained in plasma upon intravenous administration of prourokinase.

However, the high molecular weight urokinase is considered to be more effective than the low molecular weight urokinase in dissolving fibrin in vivo (literature l). Consequently, there is a strong demand for a stabilized prourokinase yielding a stabilized urokinase, which possesses the activity similar to that of naturally occurring high molecular weight human urokinase and is not susceptible to hydrolysis by proteases, and for a process for preparing the same. Such a stabilized prourokinase resulting from the conversion of amino acids as well as a process for preparing the same are unknown.

Human prourokinase has been isolated as a single chain polypeptide having a molecular weight of about 54,000 (literatures 2 and 3). Grewich et al have reported (literature 4) that when the prourokinase is converted to urokinase in vivo upon administration, its thrombolytic activity is stronger than that of a direct administration of urokinase and its ability to cause fibrinogenolysis, i.e., side effects, is reduced, suggesting its potential as a new thrombolytic agent. Since this activation of prourokinase administered is achieved by a small amount of plasmin adsorbed by blood clots at the limited site where they are present, in other words, because prourokinase is clot-specifically activated, the prourokinase is considered not to be susceptible to inhibitors in the blood stream and to provide less side effects.

However, this activation is very liable to occur, and once the prourokinase has been activated into urokinase, its degradation is rapidly increased by its own enzymatic activity. As the result the thrombolytic activity in vivo can not be sustained for a lengthy period of time. Furthermore, when the aforesaid activation has occurred during the preparation process of prourokinase, the prourokinase is broken down to a high molecular weight urokinase, which is further broken down to a low molecular weight urokinase, resulting in an increase in the amount of low molecular weight urokinase contained in the product (literature 5).

A new type of human prourokinase which is relatively nonsusceptible to activation, and thereby free from the aforesaid defect of the naturally occurring human prourokinase is therefore in demand. Such an improved human prourokinase is nevertheless unknown.

2

Note, urokinase has been prepared by extraction and purification from human urine. This process, however, is far from being industrially superior because of the problem of a stable supply of human urine. On the other hand, a process for preparing urokinase by separating human kidney tissue cells and subjecting them to passage culture has been developed of late (literature 29). It has been reported, however, that in this tissue culture the production of therapeutically effectual high molecular weight urokinase declines during the passage while the production of low molecular weight urokinase increases (literature 6). As a practical process for preparing urokinase, it is far from ideal.

Japanese Unexamined Patent Publication 59-51300 discloses a process for preparing urokinase using genetic engineering techniques. However, the method in which the expression of urokinase has been actually verified is one that comprises expressing a fused protein of trpE and urokinase by joining together the gene coding for urokinase and the trpE gene and then cutting the fused protein to obtain the low molecular weight urokinase. As such, the direct expression of prourokinase has not been verified. Further, the amount of expression of said fused protein has not been shown.

As mentioned above, a process for economically preparing prourokinase through its expression at a high efficiency is unknown.

Therefore, this invention provides a stabilized human prourokinase-like polypeptide, which is not only not susceptible to hydrolysis by various enzymes both in vitro and in vivo, but also is capable of being readily prepared as a high purity product and has a high thrombolytic specificity upon administration to the organism and an improved sustainability on the thrombolysis, a gene system for the preparation of said polypeptide, and a process for preparing said polypeptide using this gene system.

DISCLOSURE OF THE INVENTION

The present invention therefore provides a stabilized human prourokinase-like polypeptide having the amino acid sequence, or one functionally equivalent to that, of the formula :

$$\begin{array}{ccc} 1 & 135 & 157 \\ (\text{Met}) \text{ —— Ser } \text{—— X } \text{—— } \text{ Y ——} \end{array}$$

wherein Met is methionine optionally present ; Ser is the first-positioned N-terminal serine ; X is either the I35th-positioned lysine or amino acid other than basic amino acid ; Y is either the I57th-positioned phenylalanine or acidic amino acid ; and the solid line represents the amino acid sequence identical to the corresponding parts of the amino acid sequence of naturally occurring human prourokinase, with the proviso that where X is lysine, Y is not phenylalanine.

The present invention also provides the DNA which codes for said stabilized human prourokinase-like polypeptide and is capable of efficient expression in E. coli.

The present invention further provides plasmids containing said DNA, a control region necessary for the expression of said prourokinase-like polypeptide, and the DNA sequence necessary for replication in E. coli.

The present invention further provides E. coli transformed by said plasmid.

The present invention further provides a process for preparing said human prourokinase-like polypeptide using said E. coli.

BRIEF DESCRIPTION OF THE DRAWINGS

Figure I is a diagram of the plasmids of the invention ;
Fig. 2 is a restriction enzyme map for plasmid pPE3 ;
Fig. 3 shows the construction of plasmid pKYU22 from plasmid pPE3 and plasmid pKYU2I ;
Figures 4-I to 4-3 show the nucleotide sequence of cDNA from naturally occurring mRNA containing the entire coding region for human prourokinase ;
Fig. 5 shows the construction of plasmid pKMUI from plasmid pKYU22 and synthetic DNA oligomer ;

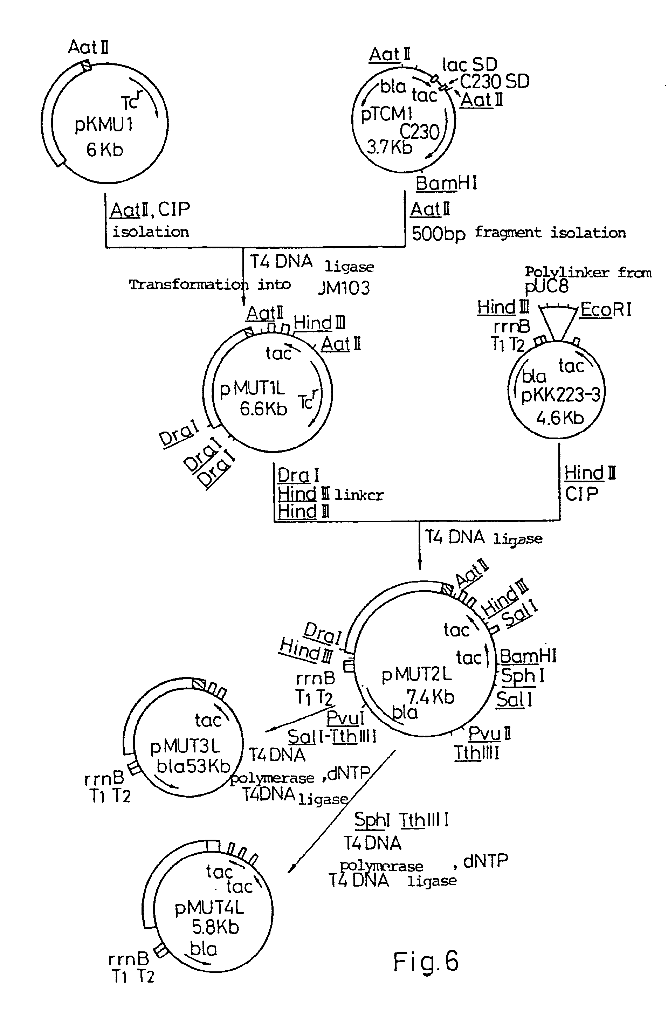

Fig. 6 shows the process of construction of plasmid pMUT4L from plasmid pKMUI and plasmid pTCMI ;
Fig. 7 shows the construction of single-stranded phage DNA with a I200 bp insert from plasmid pKYU22 and DNA of phage MI3pm8RF ;
Fig. 8 schematically shows the method of site-directed mutagenesis of a codon for amino acid I35 ;
Fig. 9 shows the construction of plasmid pMUPI from plasmids pYTUI and pKMUI ;
Fig. I0 shows the construction of plasmid pMUPIpm from the mutant phage MI3 double-stranded DNA(pmI)

and plasmid pMUPI ;
Fig. II shows the construction of plasmid pKKtrp from plasmids pSTNI6 and pKK223-3 ;
Fig. I2 shows the construction of plasmid ptrpUK2 from plasmids pKKtrp and pMUT4Lpml ;
Figures I3, I4 and I6 are the fibrin autographs of urokinase expressed in E. coli ;
Fig. I5 is a diagram comparing naturally occurring prourokinase and mutant (pm3) prourokinase of the present invention for activation by plasmin ;
Fig. I7 shows the construction of plasmid pYTUI from plasmid pHT3.

## BEST MODE FOR CARRYING OUT THE INVENTION

A. Stabilized Human Prourokinase-like Polypeptide

The stabilized human prourokinase-like polypeptide of the present invention has the amino acid sequence, or one functionally equivalent to that, of the formula :

$$
\begin{array}{ccc}
1 & 135 & 157 \\
(\text{Met}) - \text{Ser} - X - Y -
\end{array}
$$

wherein Met is methionine optionally present ; Ser is the first-positioned N-terminal serine ; X is either the I35th-positioned lysine or amino acid other than basic amino acid ; Y is either the I57th-positioned phenylalanine or acidic amino acid, and the solid line represents the amino acid sequence identical to corresponding parts of the amino acid sequence of naturally occurring human prourokinase, with the proviso that where X is lysine, Y is not phenylalanine.

Urokinase is a protein consisting of 4I0 amino acids whose sequence is well-known (literatures 8 and 9). Although the plasminogen activator-activity of urokinase is known to come from its peptide segment consisting of 275 C-terminal amino acids, it may be noted that peptides consisting solely of such segments have a low affinity for plasminogen (literature 7). The peptide consisting solely of said active segment is designated as the low molecular weight urokinase, and the urokinase consisting of 4I0 amino acids is designated as the high molecular weight urokinase. As mentioned above, the low molecular weight urokinase is thought to have a low affinity for plasminogen, and for this reason, urokinase preparations with a high content of high molecular weight urokinase are in great demand.

Consequently, nonsusceptibility to dissociation to a low molecular material is preferred for prourokinase which is a precursor of urokinase. The present inventors have found the striking fact that replacing the lysine positioned I35th from the first N-terminal serine of prourokinase with amino acids other than basic amino acid makes the cleavage between the I35th-positioned lysine and the I36th-positioned lysine (this corresponds to the change from the high molecular weight urokinase to the low molecular weight one in urokinase) difficult to occur and that such a modified polypeptide with the substituted amino acid has physiological properties similar to that of naturally occurring prourokinase (the property of activated urokinase to exhibit activity).

In the foregoing general formula for the stabilized prourokinase-like polypeptide of the present invention, X is either lysine or an amino acid other than basic amino acid, but when X is lysine, Y is not phenylalanine, as will be discussed in detail later. As the amino acid other than basic amino acid for X, any neutral amino acid or acidic amino acid which does not adversely affect the physiological property of the desired polypeptide may be used and, for example, alanine, asparagine, aspartic acid, glutamine, glutamic acid, phenylalanine, glycine, isoleucine, leucine, methionine, serine, threonine, valine, tryptophan, tyrosine, and proline may be cited. As a definite example, these may be classified to : asparagine or glutamine which is an amide of acidic amino acid; serine or threonine which is hydroxyamino acid ; glutamic acid or aspartic acid which is acidic amino acid ; and isoleucine, leucine or valine which is neutral branched-chain amino acid. The present inventors actually prepared peptides in which the I35th-positioned lysine of human prourokinase has been replaced with glutamine, threonine, glutamic acid, or isoleucine and investigated their resistance to cleavage between the I35th and I36th lysines and physiological activities. It was verified in the investigation that all of the polypeptides not only have a high resistance to cleavage between the I35th and I36th lysines but also have the physiological property of naturally occurring prourokinase. Thus it may be reasonably concluded that the prourokinase in which the I35th lysine has been replaced by the amino acid other than basic amino acid was stabilized to various proteases which recognize the two basic amino acid and cut the bond between these two basic amino acids, and that the I35th amino acids need not necessarily be lysine for the sake of urokinase activity.

The cleavage of the peptide bond between the lysine positioned I58th and the isoleucine positioned I59th

from the first N-terminal serine of urokinase by such proteases as plasmin and trypsin activates it into urokinase. Once activated, urokinase loses its activity after having been broken down by its own proteolytic activity and dissociated to a low molecular weight species. It is therefore preferable to inhibit the aforesaid cleavage in order to obtain long-lasting urokinase activity in vivo. On the other hand, the peptide bond between the l56th arginine and the l57th phenylalanine is cleaved by thrombin and the like, causing a decline in urokinase activity.

The present inventors have found the striking fact that the aforesaid cleavage by thrombin and the like is inhibited by replacing the l57th phenylalanine with aspartic acid or glutamic acid, which is acidic amino acid, and that no loss of the physiological property of naturally occurring prourokinase results from such substitution. This may be reasonably explained by theorizing that the proteases such as plasmin and trypsin which are involved in the cleavage recognize a positive charge of the l58th lysine residue side chain and that the cleavage rate may be slowed down by introducing an acidic amino acid with a negative charge in its side chain in place of amino acid in that vicinity, e.g., the l57th phenylalanine, to inhibit the protease recognition.

In the present invention the following polypeptides were prepared as examples of combinations of the aforesaid X and Y :

| Symbol | X | Y |
|---|---|---|
| lpm | Gln | Phe |
| pm2 | Thr | Phe |
| E135 | Glu | Phe |
| I135 | Ile | Phe |
| pm3 | Lys | Asp |
| Q135D157 | GIn | Asp |
| E135D157 | Glu | Asp |

As will be described in detail later, the above different polypeptides were studied on the basis of their sensitivity to trypsin for the effect of substitution at X, and on the basis of their sensitivity to both plasmin and thrombin for the effect of substitution at Y. As a result, it was confirmed that in each case the aforesaid substitution had brought about the desired stabitizing effect and that the inherent physiological property of naturally occurring prourokinase had not been lost by these substitutions. Thus it may be reasonably assumed, coupled with the aforesaid assumed stabilization mechanism, that the polypeptides consisting of all of the combinations of previously defined X and Y do exert the stabilizing effect of the present invention. Accordingly, the prourokinase-like polypeptides consisting of the combinations of X and Y previously defined fall within the purview of the present invention.

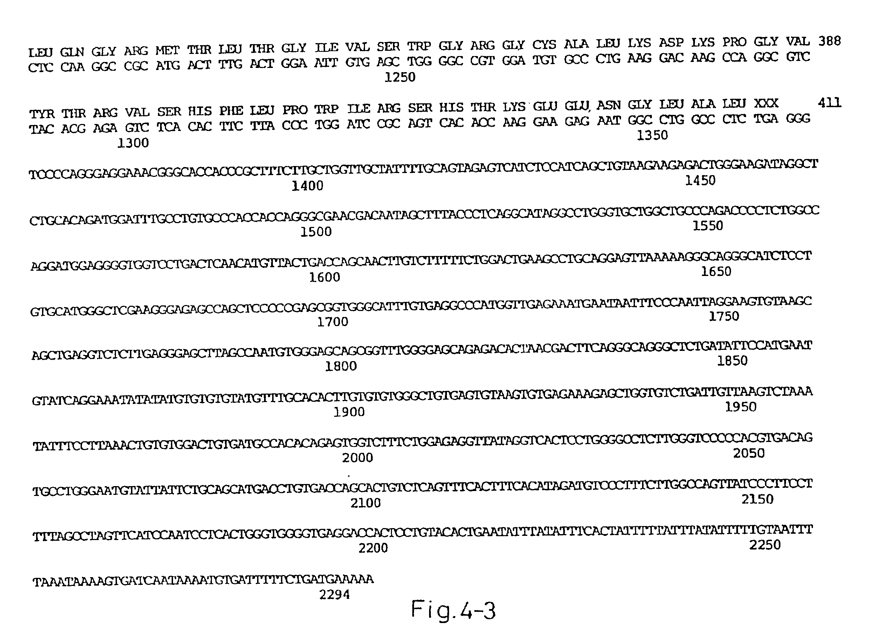

The amino acid sequences represented by the solid lines in the preceding general formula are identical to the corresponding parts of the amino acid sequence of naturally occurring human prourokinase. As an example of an amino acid sequence of naturally occurring human prourokinase, a sequence consisting of 4ll amino acids coded by cDNA corresponding to mRNA derived from a human kidney may be cited. This amino acid sequence is represented by the abbreviation for amino acid consisting of three capital letters of the alphabet, in Figures 4-l to 4-3.

In the foregoing general formula (Met)-represents methionine which would exist adjacent to the first N-terminal Ser and on its N-terminal side. According to the preparation process of the present invention, which will be explained in detail later, a polypeptide containing methionine derived from the translation initiation codon is expressed at the N-terminus, but a polypeptide without any methionine attached to the N-terminus would be obtained by the removal of methionine by enzymes within E. coli cells. Accordingly, the present invention includes both polypeptides with and without the methionine.

The stabilized human prourokinase-like polypeptides of the present invention includes not only the polypeptide containing the amino acid described in detail above but also any polypeptide containing the amino acid sequence functionally equivalent to that sequence. The term "funtionally equivalent amino sequence" as used herein means any amino acid sequence in which several amino acids other than X and Y in the amino acid sequence described above have either been replaced with other amino acids or removed, or in which several amino acids are added to the amino acid sequence described above but the physiological property of prourokinase and the stability which characterizes the present invention are still retained. The fact that there may be cases where alterations to the amino acid sequence of a polypeptide having a specific physiological activity,

5

made in the region not concerned in said physiological activity, do not affect said physiological activity is well-known to those skilled in the art. Therefore, polypeptides that embrace such alterations do fall within the purview of the present invention as long as they retain the characteristics of the present invention.

B. Gene System of Human Prourokinase

This invention further relates to a gene system which is useful for preparing stabilized human prourokinase. The gene system referred to herein includes DNA segments coding for the desired human prourokinase-like polypeptide, plasmids containing said DNA, and hosts into which such a plasmid has been introduced.

Typical DNA segments of the present invention include those in which codons coding for a plurality of N-terminal amino acids are those which are used in E. coli at high frequency and codons other than said codons coding for a plurality of amino acids, and other than codons coding for said X and Y, are identical to the codons of the corresponding amino acids in cDNA that corresponds to mRNA derived from human kidney.

As genes coding for a protein consisting of a large number of amino acids such as human prourokinase, it is of advantage to use a DNA segment derived from cells of an organism that produces such a protein in nature, that is, human cells, such as cDNA obtained by using mRNA coding for prourokinase as a template ; and even so it is not impossible to synthesize DNA coding for such high molecular weight protein, but this calls for great efforts. On the other hand, it seems preferable to use genes consisting of codons used in E. coli at a high frequency in order to effectively express the protein in E. coli, but these conditions are not necessarily fully satisfied in human-derived cDNA, leaving no alternative but to rely on chemical synthesis to obtain DNA that satisfy such conditions.

Mainly using human-derived cDNA as genes coding for human prourokinase, the present inventors obtained genes that are effectively expressed in E. coli by replacing only the codons coding for a few amino acid at the N-terminus of prourokinase in said cDNA with synthesized DNA consisting of codons used at high frequency in E. coli.

In selecting codons of said synthetic DNA segment, it is preferable to prevent the fold-back of the mRNA by eliminating the inverted repetitive sequence of 16 bases of the urokinase gene of the natural type (cDNA), and to further prevent at the mRNA level the complementary association of the nucleotide sequence in the vicinity of the SD (Shine-Dalgarno) sequence of the metapyrocatechase gene (C230) used in the preferred embodiments of expression plasmids of this invention and the nucleotide sequence at the 5′ end of the human prourokinase gene by eliminating their complementary structures. It is also preferable to select the codon for serine which is the first amino acid so that the base next to the translation initiation codon ATG will be A.

In the preferred embodiments of this invention, the direct expression of prourokinase is made possible by placing the translation initiation codon ATG coding for methionine adjacent to and upstream of the codon for serine, the N-terminal amino acid, in the coding region designed in the manner described above. In the preferred embodiments of this invention, furthermore, the following recognition sequence for the restriction endonuclease AatII :

$$5' \quad \quad C(ATG) \quad 3'$$

$$3' \quad TGCAG(TAC) \quad 5'$$

is placed adjacent to and upstream of said ATG so that on insertion of DNA of said coding region into an expression vector, the distance and structure between the SD sequence of the metapyrocatechase gene used in the preferred expression vector of the present invention and the ATG of the inserted high molecular weight urokinase gene will be identical to those in the natural metapyrocatechase gene.

In the preferred embodiments of the present invention, therefore, the DNA sequence coding for 6 N-terminal amino acids of the human prourokinase and its upstream portion of the cDNA coding for human prourokinase are altered as shown in Fig. I, and in other coding region, the DNA sequence of cDNA (mutations designed to code for amino acids X and Y are included where applicable) is used. Furthermore, the restriction endonuclease site SstI is provided in this region to enable screening of recombinants and the replacement with other DNA sequences.

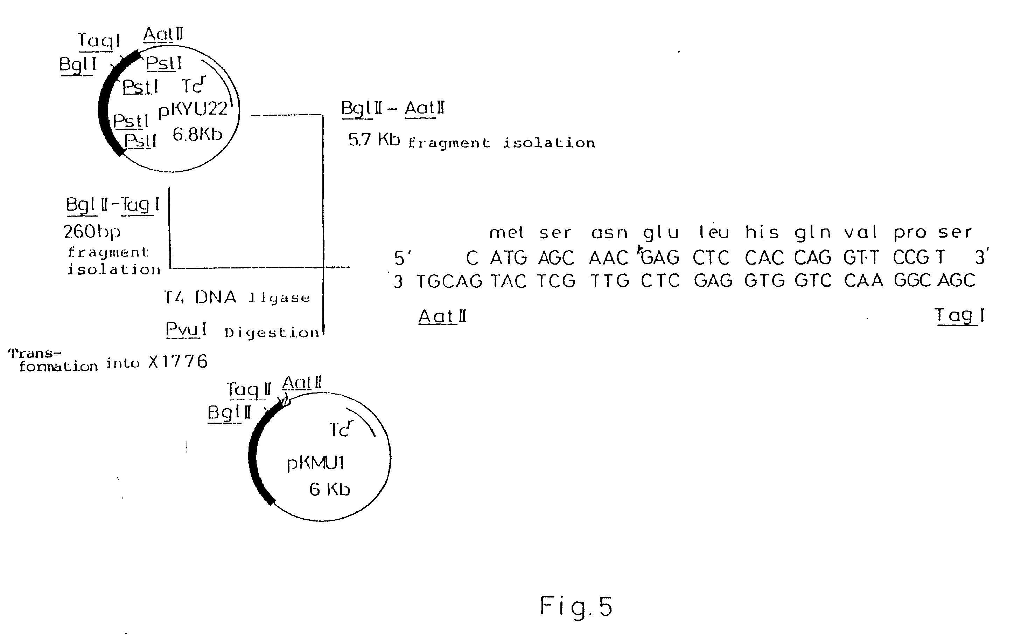

An example a of modified nucleotide sequence of the present invention is shown in Fig. 5. In this nucleotide sequence, codons used at high frequency in E. coli are used for asparagine, glutamic acid, leucine, histidine, glutamine, and proline. Further in this nucleotide sequence the leader sequence present in natural cDNA is eliminated to enable the direct expression of human prourokinase, and instead, the translation initiation codon coding for methionine is placed adjacent to and upstream of the codon for serine which is the first amino acid of human prourokinase. In addition, the AatII site is provided to permit insertion into the expression plasmid.

Where X in the general formula is the amino acid other than basic, the codon coding for a desired amino acid other than the basic amino acid must be used instead of the cDNA codon coding for lysine as X. Any codons may be used in place of the one for l35th lysine as long as they code for the corresponding amino acid and are readily expressible in E. coli. Where glutamine is used as the l35th amino acid, however, it is preferable to use the codon consisting of CAA, whereas the ACA is preferred when threonine is used. For example, the following codons may be used for respective amino acids :

| 135th Amino Acid | Codon |
| --- | --- |
| Alanine | GCA |
| Asparagine | AAC |
| Aspartic acid | GAC |
| Glutamic acid | GAA |
| Phenylalanine | TTC |
| Glycine | GGT |
| Isoleucine | ATC |
| Leucine | CTG |
| Methionine | ATG |
| Serine | AGC |
| Valine | GTA |
| Tryptophan | TGG |
| Tyrosine | TAC |
| Proline | CCG |

Where Y in the general formula is acidic amimo acid, the codon coding for acidic amino acid must be used in place of the cDNA codon coding for phenylalanine as Y. Any codons may be used other than the one for the l57th phenylalanine as long as they code for the corresponding amino acids and are readily expressible in E. coli. When aspartic acid is used as l57th amino acid, the use of the codon consisting of GAT is preferred, and when glutamic acid is used, the GAA is preferred.

(2) Plasmid

Although vectors that function in animal cells may be used for expression of the human prourokinase-like polypeptide of the present invention, the use of a plasmid containing along with said region the expression control region necessary for expressing said genes in microorganisms, particularly in E. coli and the region necessary for replication in E. coli is preferred. As the expression control region any system useful for expressing the foreign gene in E. coli may be use arbitrarily. For example, the promoter/operator systems tac, $P_L$, lacUV5, $P_R$, trp, and lpp are used. In particular, the tac promoter/operator, the $P_L$ promoter/operator, and the trp promoter operator are preferred . As an example of SD sequences, the SD sequence of the metapyrocatechase gene (C230SD) (literature l0) and lacSD may be used.

The following may be cited as plasmids capable of expressing the polypeptide of the present invention :

| Plasmid | X | Y |
|---|---|---|
| pMUPlpm | Gln | Phe |
| pMUT4Lpm2 | Thr | Phe |
| ptrpUK2(E135) | Glu | Phe |
| ptrpUK2(I135) | Ile | Phe |
| pMUT4Lpm3 | Lys | Asp |
| ptrpUK2(Q135D157) | Gln | Asp |
| ptrpUK2(E135D157) | Glu | Asp |

(3) Hosts for Plasmid Introduction

Although animal cells and such micro-organisms as bacteria and yeast may be used as hosts for introducing said plasmids, the use of bacteria, particularly E. coli, is preferred.

As host E. coli of the present invention, such strains of nonenterositic, nontoxic E. coli as those derived from E. coli K-l2 may be used, e.g., JM83, JMl03, JMl05, RB79l, SM32, N99, RRl, W3ll0, and χl776.

C. Construction of Gene System

An example of the process for constructing genes involved in the present invention comprises the following steps :

(l) Preparing DNA fragments completely coding for human prourokinase ;

(2) Causing changes in the codon coding for the l35th aspartic acid of the polypeptide of said human prourokinase to convert it to the codon coding for an amino acid other than basic amino acid ;

(3) Causing changes in the codon coding for the l57th amino acid of the polypeptide of said human prourokinase to convert it to the codon coding for acidic amino acid ;

(4) Joining together any two kinds of DNA fragments prepared in said steps (l), (2) and (3) to prepare DNA fragments coding for the desired stabilized human prourokinase-like polypeptide ;

(5) Replacing a part of said DNA segment coding for a few amino acids at the N-terminus of said polypeptide with a DNA fragment consisting of codons that code for the same amino acid and consist of codons readily expressible in E. coli.

Of said steps (l) ~ (5), step (l) is critical, but steps (2) and (3) may be carried out electively according to the type of the desired human prourokinase-like polypeptide.

These steps can be carried out in any order. In order to obtain genes showing efficient expression it is preferable to carry out step (5) and the priority in carrying out this step may be decided on an arbitrary basis.

Of course the DNA segment of the gene system of the present invention can be totally synthesized. In this instance it is possible to use codons that are readily expressible in E. coli, instead of natural codons.

A diagram for constructing various plasmids involved in the present invention is shown in Fig. l and the definite methods of construction are described in the examples and reference examples of the present invention.

D. Expression of Gene and Characterization of Expression Products

Plasmid constructed in the manner described above is transformed to an appropriate host such as E. coli JMl03 or W3ll0, and the transformant incubated in a suitable medium such as L-medium which contains 50 μg/ml of ampicillin, as the case may be, and gene expression induced when the growth of E. coli has reached a given level, e.g., absorbance of 0.3 to 0.5 at 550 nm. The induction procedure varies with the type of promoter of the plasmid used. For example, where the plasmid pMUPlpm is used, E. coli cells transformed with said plasmid are incubated at 30°C and the gene expression is induced by increasing the incubation temperature to 42°C. Where the plasmid pMUT4Lpm2 or the plasmid pMUT4Lpm3 is used, the induction is carried out with isopropyl-β-D-thiogalactopyranoside. Where the plasmid ptrpUK2(Il35), the plasmid ptrpUK2(El35), the plasmid ptrpUK2(Ql35Dl57)or the plasmid ptrpUK2(El35Dl57) is used, the induction can be carried out by adding indoleacetic acid when the cell concentration has reach a given level, e.g., absorbance of 0.3 to 0.5 at 550 nm.

After gene expression has been thus induced the incubation is carried out further for 3 to l0 hours to produce the desired polypeptide. Then the cells are collected and a cell-free extract containing the desired polypeptide is obtained by the conventional method, e.g., the method described in detail later in Examples of the present invention. If desired, the extract is purified to yield a prourokinase-like polypeptide by affinity chromatography using a Sepharose® column in which antiurokinase monoclonal antibodies are held.

In the present invention the extract obtained as described above was tested for enzyme activity, molecular weight, trypsin sensitivity, plasmin sensitivity, and thrombin sensitivity.

## (l) Measurement of Enzyme Activity

Synthetic Substrate Method :

Seven hundred μl of 0.2 mM S-2444 (L- pyroglutamyl-glycyl-L-arginine P-nitroanilide, Kabi & Co., Sweden), 0.l M Tris-HCl (pH 7.5), and 0.0l% Triton® X-l00 is added to l00 μl of a sample and the mixture is incubated for 30 minutes at 37°C. The reaction is stopped by addition of l00 μl of acetic acid and then the absorbance is measured at 405 nm.

Fibrin Plate Method :

Ten μl of a sample is spotted on a fibrin plate prepared by the addition of thrombin to 67 mM phosphate buffer containing l% bovine fibrinogen (95% clottable, made by Miles Inc.) and 0.25% agarose, to make the final concentration l unit/ml. After incubation for l6 hours at 37°C, the activity is assayed by measuring the area of the dissolved ring and comparing the same with the standard urokinase (made by Nippon Soda Co.).

## (2) Measurement of Molecular Weight

A sampled extract is separated by SDS polyacryl amide gel electrophoresis under the condition free of 2-mercaptoethanol using the method of Laemmli et al (literature 30) and compared with the standard sample after being made visible by fibrin autography.

## (3) Trypsin Sensitivity

After treating a sample with trypsin, the molecular weight of the enzymatically active product in the sample is measured by the method described in Paragraph (2) above. The stability of the urokinase obtained from the substitution of the l35th amino acid is confirmed by this testing.

## (4) Plasmin Sensitivity

The enzyme activity of a sample is measured by the fibrin plate method and the sample is diluted with a solution containing 50 mM veronal buffer, 0.l M NaCl, and 0.00l% Tween® 80, to make the final concentration 500 IU/ml. One μg, 2 μg, and 5 μg portions of plasmin are added respectively to l00 μl aliquots of the solution and incubated at 37°C. Ten μl each of the samples are withdrawn with the passage of time, and after the addition of 5 μg of soybean trypsin inhibitor, the reaction is stopped and the activity is measured by the synthetic substrate method using S-2444. The nonsusceptibility of prourokinase to plasmin treatment by virtue of its activation on cleavage between the l58th lysine and the l59th isoleucine by plasmin serves as an indication of the stability of the prourokinase-like polypeptide obtained through substitution of the l57th amino acid.

## (5) Thrombin Sensitivity

The enzyme activity of a sample is measured by the fibrin plate method and the sample is diluted with a solution containing a 50mM veronal buffer, 0.l M NaCl, and 0.00l% Tween® 80, to make the final concentration 50 IU/ml. One ml of the solution is added with l unit of thrombin and incubated for 30 minutes at 37°C. One hundred μl of a sample is withdrawn and the activities are assayed by the synthetic substrate method using S-2444 after treatments with l μg of plasmin for 30 minutes at 37°C for the enzyme of the natural type and with 5 μg of plasmin for 60 minutes at 37°C for the mutant enzyme. The residual activity after thrombin treatment is assayed using as a control the sample obtained after carrying out the same manipulation without thrombin. The activity of prourokinase decreases when a bond between the l56th arginine and the l57th phenylalanine is severed by thrombin. Accordingly, the absence of decline in the residual activity after thrombin treatment is an indication of the stability of the prourokinase-like polypeptide obtained through substitution of the l57th amino

acid.

Results

(I) Enzyme Activity

Each sample obtained from E. coli transformed with the plasmid of the present invention exhibited the desired activity on measurement of enzyme activity carried out in the manner described in the preceding Paragraph (I).

(2) Trypsin Sensitivity

The samples prepared from E. coli transformed with any of the plasmid such as plasmid pMUPI coding for prourokinase-like polypeptide in which neither the I35th amino acid nor the I57th amino acid has been substituted ; plasmids pMUPIpm, pMUT4Lpm2, ptrpUK2(II35), and ptrpUK2(EI35) containing the genes coding for the prourokinase-like polypeptide in which only the I35th amino acid has been substituted ; plasmids ptrpUK2-(QI35DI57) and ptrpUK2(EI35DI57) containing the genes coding for the prourokinase-like polypeptide in which both the I35th amino acid and the I57th amino acid have been substituted, exhibited resistance to trypsin treatment except for the samples derived from plasmid pMUPI.

For example, E. coli W3II0 was transformed by each of plasmids pMUPI and pMUPIpm, the transformants cultured in L-broth at 30°C, and the gene expressed by increasing the incubation temperature to 42°C when the O.D. level at 600 nm has reached 0.3.

The cells were collected, from which cell-free extracts were obtained. The extracts were assayed for the molecular weight by SDS polyacrylamide gel-fibrin autography with each of the extracts assaying approximately 50,000 (Lanes 2 and 4 in Fig, I3). After trypsin treatment the extracts were assayed for the molecular weight by the same method with the result that a product having a molecular weight of about 30,000 was found to be present in the extract derived from plasmid pMUPI while very little of such product was present in the extract derived from plasmid pMUPIpm (Lanes 3 and 5 in Fig. I3).

It is evident from the above result that the product derived from plasmid pMUPI is readily converted to low molecular weight species by trypsin while such conversion to low molecular weight species does not take place with the product of the present invention.

E. coli JMI03 was likewise transformed by plasmid pMUT4Lpm2 and the transformant was incubated in L-broth until the absorbance at 550 nm reached approximately 0.5. After the addition of an IPTG inducer (isopropyl-β-D-thiogalactopyranoside) to make the final concentration I mM, the incubation was further carried out at 37°C for gene expression. The cells were treated in the same manner as described above and the product was analyzed also in the same manner as described above.

The results are shown in Fig. I4. The lane I in the figure represents the standard urokinase with the lane 2 representing the result of trypsin treatment of the crude extract derived from E. coli cells transformed by plasmid pMUPI, the lane 3 representing the result of trypsin treatment of the crude extract derived from E. coli cells transformed by plasmid pMUT4Lpm2, and the lane 4 representing the result of trypsin treatment of the crude extract of E. coli cells transformed by plasmid pMUPIpm. As is evident from these results, the gene product derived from plasmid pMUT4Lpm2 is also not liable to conversion to low molecular weight species by trypsin.

The aforesaid products having molecular weight of about 50,000 and about 30,000 are thought to correspond respectively to the high molecular weight urokinase and the low molecular weight urokinase. The molecular weight of these products being slightly lower than that the standard urokinase derived from human urine is presumably attributable to the failure of the addition of glycoside chains within the E. coli cells.

Universality of Amino Acid I35 Substitution

The above results clearly show that the human prourokinase-like polypeptides which have virtually the same amino acid sequence wherein the I35th lysine calculated from the N-terminal serine has been replaced with glutamine, threonine, glutamic acid, or isoleucine exhibit the fibrinolytic activity, i.e., urokinase activity, similar to that of naturally occurring human prourokinase and that, unlike the naturally occurring human urokinase, they are not susceptible to degradation by trypsin.

This justifies the assumption that the formation of low molecular weight urokinase results from the cleavage of the peptide bond between the I35th and I36th basic amino acids (lysine) calculated from the N-terminal serine of high molecular weight urokinase by trypsin-like protease. Furthermore, the fact that said cleavage is less liable to occur when N-terminal amino acid I35 is the above-mentioned amino acid means that when the amino

acid I35 is any amino acid other than a basic amino acid the peptide bond between amino acids I35 and I36 is not susceptible to cleavage.

Moreover, the fact that urokinase activity is generated whether the N-terminal amino acid I35 is lysine as in naturally occurring human urokinase or the above-mentioned amino acid as in the human urokinase-like polypeptide of the present invention shows that urokinase activity is irrelevant to what amino acid I35 is used.

Since numbers of protease are said to recognize basic amino acids, if the I35th amino acid calculated from N-terminal amino acid is an amino acid other than a basic amino acid, the same result as that of the polypeptide disclosed definitely in this specification is likely to be obtained.

(3) Plasmin- and Thrombin-Sensitivities

The samples prepared from E. coli transformed by any of the three plasmids, e.g., plasmid pMUT4Lpm3 containing the gene coding for the prourokinase-like polypeptide in which only the amino acid I57 has been replaced, and plasmids ptrpUK2 (QI35DI57) and ptrpUK2 (EI35DI57) both containing the gene coding for the prourokinase-like polypeptide in which both the amino acids I35 and I57 have been replaced showed the activation by plasmin slower than prourokinase of the natural type. One example of this is graphically shown in Fig. I5.

As is evident from this graph the prourokinase-like polypeptide of the present invention in which the amino acid I57 has been replaced is not susceptible to plasmin degradation (activation).

The residual enzyme activities after thrombin treatment were as follows :

| Prourokinase | Residual Activity after Thrombin Treatment |
|---|---|
| Natural-type Prourokinase | 36% |
| Mutant (pm3) Prourokinase | 107% |
| Mutant (Q135D157) Prourokinase | 105% |
| Mutant (E135D157) Prourokinase | 98% |

The above result shows that the prourokinase-like polypeptides in which the amino acid I57 has been replaced are not susceptible to thrombin inactivation.

It is thus evident that the human prourokinase-like polypeptide in which the lysine I35 has been replaced with an amino acid other than a basic amino acid, the human prourokinase-like polypeptide in which the phenylalanine I57 has been replaced with an acidic amino acid, and the human prourokinase-like polypeptide in which both amino acids have been replaced as described above are all physiologically active as is human prourokinase of the natural type within the purview of the amino acids defined in the present invention, and in addition, are quite stable to such proteases as trypsin, plasmin, and thrombin.

This suggests that the stabilized human prourokinase-like polypeptide of the present invention may be a promising active ingredient for pharmaceuticals such as a thrombolytic agent and the like.

The following reference examples and examples are illustrative of the invention but are not to be construed as limiting.

Reference Example I.

Preparation of mRNA

mRNA was prepared by the method of J.M. Chirgwin et al (literature II) using guanidine thiocyanate.

Twenty g of kidney tissue cells frozen at -80°C was crushed with a Waring blender in liquid nitrogen and suspended in 80 ml of a 5 M guanidine thiocyanate solution (5 M guanidine thiocyanate, 0.5% sodium N-lauroylsarcosine, 25 mM sodium tartrate, 0.I M melcaptoethanol, and 0.I% antifoam A). The suspension was homogenized with a Teflon homogenizer and nucleic acid was shared with a 20G I/2 injection needle. Twenty-four ml of said solution was layered over I2 ml of 5.7 M CsCl and after centrifugation with a Beckman centrifuge in SW28 rotor for 24 hours at I5°C and at 25,000 rpm the whole crude RNA was recovered.

The total crude RNA was dissolved in 2% potassium acetate solution and twice its volume of ethanol was added. The mixture was allowed to stand overnight at -20°C and centrifuged to recover precipitate.

In accordance with the method of H. Aviv et al (literature 12), poly(A)+ RNA was isolated and purified by oligo (dT) cellulose column chromatography. Ten g of the kidney tissue cells yielded about 3 mg of the total RNA, of which 2 to 3% was poly(A)+ RNA.

## Reference Example 2.

### Construction of cDNA Library (I)

cDNA synthesis was carried out using 40 µg of the poly(A)+ RNA obtained in Reference Example I. Using 40 µg of oligo (dT)$_{12-18}$ as a primer, reaction was carried out with 40 units of reverse transcriptase for 2 hours at 42°C to synthesize the first chain and after removal of the template mRNA by alkali treatment the synthesis of the second chain was carried out with 100 units of E. coli DNA polymerase I Klenow fragment.

After elimination of the hairpin loop with SI nuclease the (dC)$_{10-20}$ chain was bonded to the 3' end of the double-stranded cDNA with terminal deoxynucleotidyl transferase and about 400 ng of (dC) tailed cDNA was obtained.

This material was annealed together with 800 ng of a commercially available (dG) tailed pBR322 (PstI site) (made by New England Nuclear Inc.) and E. coli XI776 was transformed by the Hanahan method (literature 13). A cDNA library (I) consisting of about $2 \times 10^5$ tetra-cycline-resistant and ampicillin-sensitive transformants was thus obtained.

The size of cDNA insertion fragments was determined by the rapid isolation method (literature 14) using the alkali lytic procedure on these transformants.

## Reference Example 3.

### Preparation of Synthetic DNA

### Oligomers for Use as cDNA Library Screening Probes

The following 16 different DNA oligomers consisting of 14 nucleotides which are complementary to the mRNA corresponding to the amino acid sequence of the human urokinase Asn[169], Gln, Pro, Trp, Phe[173] which has been reported by G.J. Sfeffens et al (literature 8) and Gunzler et al (literature 9) were synthesized by the phosphotriester method :

```
AACCAAGGTTGATT
AACCAAGGTTGGTT
AACCAGGGTTGATT
AACCACGGTTGATT
AACCACGGTTGGTT
AACCATGGTTGATT
AACCATGGTTGGTT
AACCAAGGCTGATT
AACCAAGGCTGGTT
AACCAGGGCTGATT
AACCAGGGCTGGTT
AACCACGGCTGATT
AACCACGGCTGGTT
AACCATGGCTGATT
AACCATGGCTGGTT
AACCAGGGTTGGTT
```

These l6 different DNA oligomers are referred to hereinafter as UK probe I.

For use as confirmatory probes, the following 8 different DNA oligomers consisting of l4 nucleotides which are complementary to the mRNA corresponding to Met[283], Try, Asn, Asp, Pro[287] were synthesized in the same manner.

```
5' GGATCATTATACAT 3'
   GGATCGTTATACAT
   GGATCGTTGTACAT
   GGATCATTGTACAT
   GGGTCATTATACAT
   GGGTCGTTATACAT
   GGGTCGTTGTACAT
   GGGTCATTGTACAT
```

These 8 different DNA oligomers are referred to hereinafter as UK probe II.

By use of T4 polynucleotidekinase, 200 ng each of the UK probes I and II was radioactively labeled at the 5'end with 3000 Ci/mmole $^{32}$P$\gamma$ATP to prepare probes for colony hybridization.

Reference Example 4.

Screening of cDNA Library (I)

(I) Screening

An autoclaved nitrocellulose filter (0.45 μm, Type-TM-2 made by Toyo Roshi Co.) was placed on an LB agar medium containing l5 μg/ml of tetracycline and the medium was inoculated with the transformants prepared in Example 2 so as to allow about 2,000 transformant colonies to grow. After 8 hours of incubation at 37°C the colonies were replicated on two nitrocellulose filters, which were incubated further for 3 hours at 37°C. The original nitrocellulose filter was used as a master filter, while two second filters were transferred to LB agar media containing l5 μg/ml of tetracycline and l00 μg/ml of chloramphenicol and were subjected to overnight incubation at 37°C. In accordance with the modified method of Grunstein and Hogness (literature I5) the filters were then placed over 0.5 M NaOH and I.5 M NaCl for 3 minutes to effect colony lysis and DNA denaturation, and after neutralization over 0.5 M Tris-HCl (pH 7.6) and I.5 M NaCl, were air-dried and baked for 2 hours at 80°C.

After washing the filters in 4 x SSC for 30 minutes each at 60°C, prehybridization was carried out in 4 x SSC, I0 x Denhardt, and 50 μg/ml denatured E. coli-DNA for one hour at 60°C, and after addition of 0.I mM ATP and the radioactively labeled UK probe I (about I0⁷ cpm/filter), hybridization was carried out for I6 hours at 37°C. After washing 6 to 8 times in 4 x SSC at 39°C, the filters were air-dried and screened for transformants hybridizing with the UK probe I to obtain 2I candidate clones from 8 x I0⁴ colonies.

Plasmids of these clones are hereinafter referred to as plasmids pKYUI to pKYU2I.

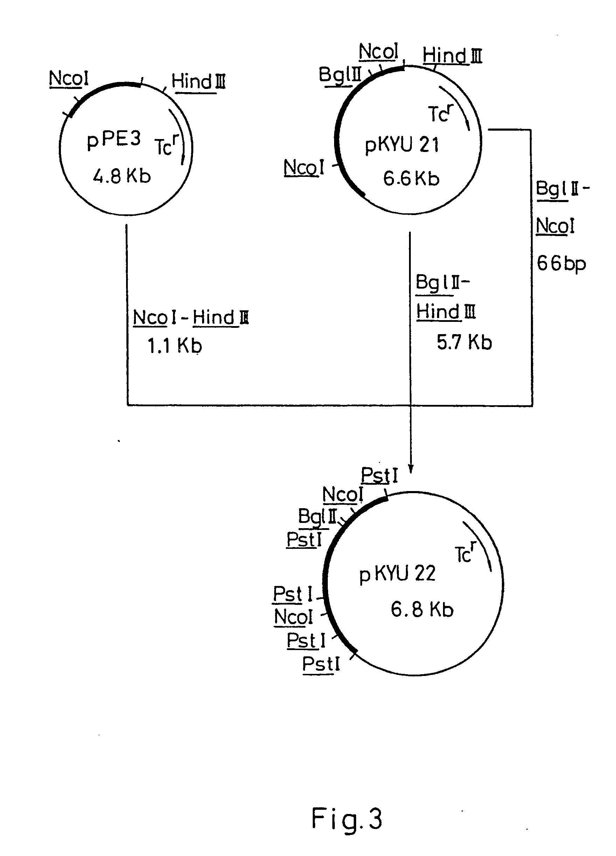

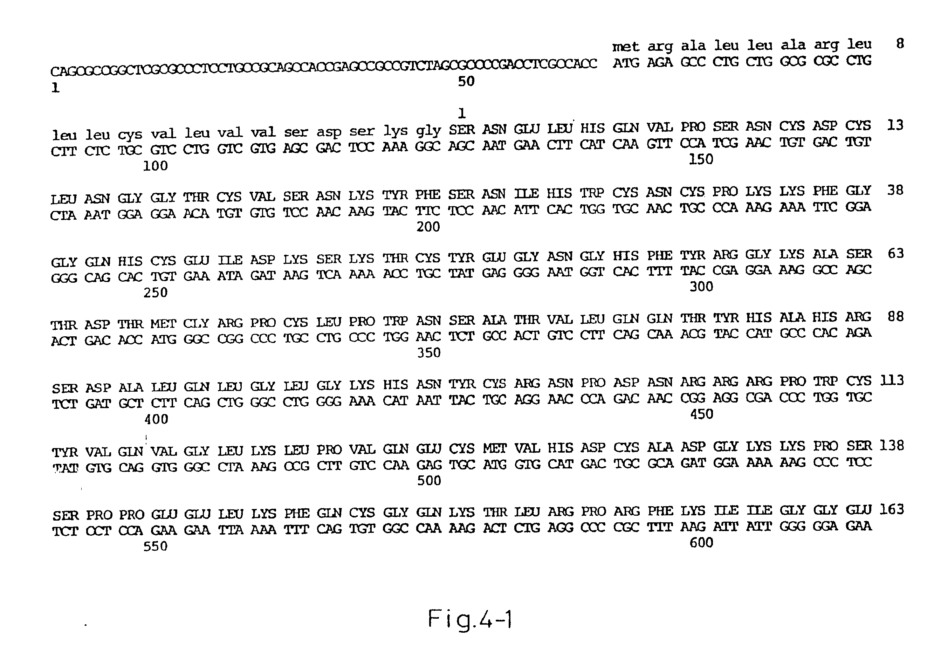

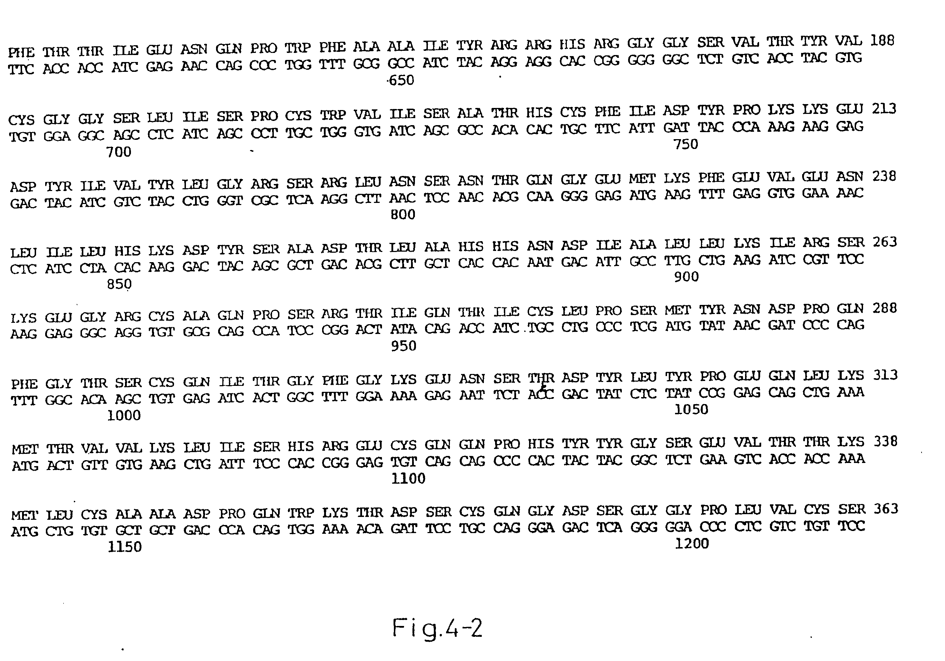

The 2I candidate clones obtained were treated in the same manner as described above and clones hybridizing with the UK probe II were obtained. The plasmids in these clones are hereinafter referred to as plasmid pKYU2 (Fig. 3).

(2) Characteristics of pKYU2I Plasmid DNA

After digesting the pKYU2I plasmid DNA with various restriction endonucleases and subcloning them to MI3mp8 by the Maxam-Gilbert method (literature I6), determination of the nucleotide sequence was carried out by the dideoxy chain termination method (literature I7). On contrasting this sequence with the well-known amino acid sequence of urokinase it was confirmed that although the cDNA contains the entire code region of low molecular weight urokinase, about I00 bp long DNA in the 5'-end region of the coding region of high molecular weight urokinase is lacking.

Reference Example 5.

Construction of cDNA Library (II) by Primer Extension Reaction

Based on the nucleotide sequence determined in Paragraph (2) of Reference Example 4, the following DNA oligomer:

$$\text{5' CTGAAGAGCATCAGA 3'}$$

consisting of I5 nucleotides which are complementary to the mRNA sequence corresponding to [89]Ser, Asp, Ala, Leu, Glu[93] was synthesized by the phosphotriester method.

Employing I00 μg of poly(A)⁺ RNA as a template and complying with the method of Agarwall et al (literature I8) or the method of Stewart et al (literature I9), the first cDNA chain was synthesized using I00 units of reverse transcriptase together with I μg of 5' ³²P-labeled primer followed by the synthesis of the second chain with I00 units of E. coli DNA polymerase I Klenow fragment. Then the single-stranded DNA was digested with SI nuclease and (dC)n chain was added at the 3'-end using terminal deoxynucleotidyl transferase. After annealing this dC tailed insert (cDNA) and a dG tailed vector (pBR322) together, E. coli XI776 was transformed to obtain a cDNA library (II) consisting of about 5 x I0⁴ transformants.

Reference Example 6.

Screening of cDNA Library (II)

The transformants obtained in Reference Example 5 were subjected to hybridization in the same manner as that described in Paragraph (I) of Reference Example 4. In this case, a I50 bp PstI-BgIII 5' digestion fragment from pKYU2I radioactively labeled by the nick translation method (Literature I3) using α³²PdCTP (3000 Ci-/mmole) was used as a probe. The hybridization was carried out at 60°C.

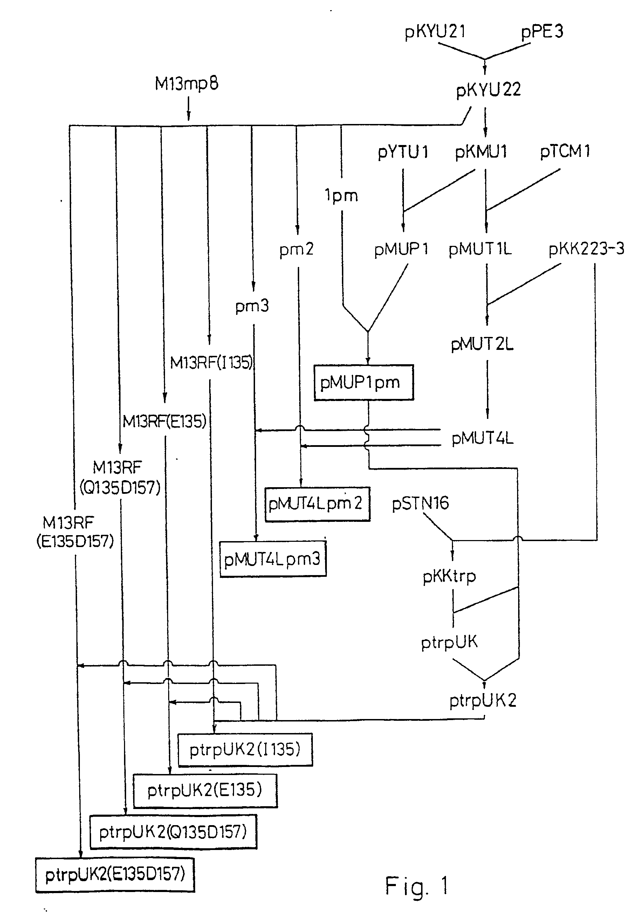

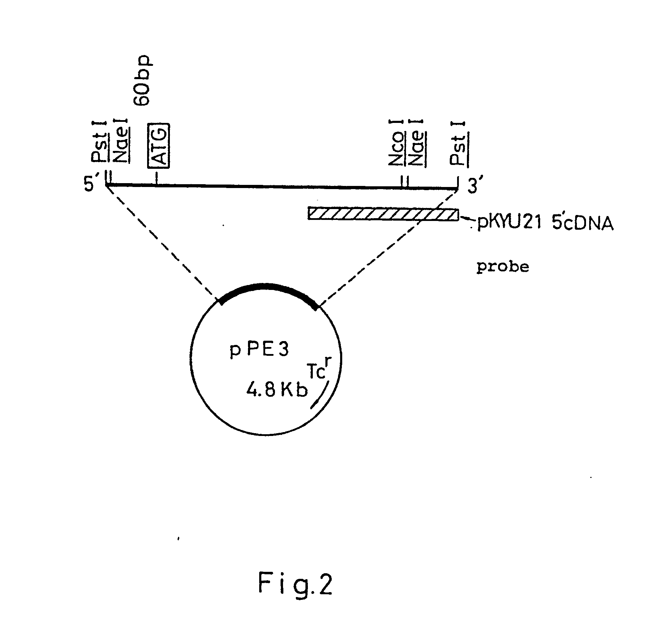

The filter was air-dried after washing two or three times with 2 x SSC at 60°C, and clones hybridizing with said probe were retrieved by autoradiography. Eight positive clones were obtained out of about 3 x I0⁴ clones. The plasmids in these clones are hereinafter referred to as Plasmids pPEI to pPE8. On digestion of these plasmid DNAs with restriction enzyme PstI it was confirmed that Plasmid pPE3 (Fig. 2) contains an approximately 420 bp long cDNA insert.

The fragments obtained through digestion of DNA of plasmid pPE3 with restriction endonuclease PstI were subcloned into MI3pm8, and determination of the base sequence was carried out by the dideoxy chain termination method (literature I7). As the result, it was confirmed that the cDNA contains not only a sufficiently long coding region on the 5'-end side of the prourokinase gene but also a 66 bp long 5'-nontranslation region upstream of the translation initiation codon ATG (Fig. 2).

Reference Example 7.

Construction of Prourokinase Gene (Fig. 3)

Five µg of DNA of plasmid pKYU2l containing an entire coding region of the low molecular weight urokinase was digested twice with l0 units each of restriction endonucleases BglII and HindIII and then electrically eluted to give about 5.7 Kbp DNA fragment, while DNA of said plasmid pKYU2l was digested twice with l0 units each of BglII and NcoI and then eluted to give a 66 bp DNA fragment. Then again 5 µg of DNA of plasmid pPEP3 obtained in Reference Example 6 was digested twice with l0 units each of NcoI and HindIII to give about l.l Kbp DNA fragment. These three different DNA fragments were repeatedly extracted with phenol/chloroform, precipitated with 2 volumes of ethanol and the precipitate recovered and purified. These three different DNA fragments were ligated together with T4DNA ligase for transformation into E. coli χl776. The resultant transformants were screened by the rapid isolation method using the alkali lysis procedure and a clone carrying plasmid pKYU22 that contains the entire gene for prourokinase was obtained.

The clone, E. coli χl776/pKYU22 was deposited on January ll, l985 with Fermentation Research Institute, Agency of Industrial Science and Technology.(l-3, Higashi l-chome, Yatabe-cho, Tsukuba-gun, Ibaraki-ken, Japan) as FERM P-804l and on January 22, l986 was placed under international deposition as FERM BP-968 pursuant to the provisions of the Budapest Treaty.

Reference Example 8.

Nucleodide Sequence Determination of Plasmid pRYU 22 Insertion Site

The nucleotide sequence at the insertion site of plasmid pKYU22 was determined by the Maxam-Gilbert method, and the dideoxy chain termination method preceded by subcloning to Ml3mp8. The result is presented in Figures 4-l to 4-3. Comparison of this result with the nucleotide sequence described in literature 20 revealed the following discrepancies : codon AAT of 254th amino acid Asn as against AAC in the literature ; codon CTG of 360th amino acid (the number of amino acid in Fig. 4-2) Leu as against CTA in the literature ; codon CCA of 365th amino acid Pro as against CCC in the literature ; and codon CAG of 366th amino acid Gln as against CAA. While codon AAT is said to be difficult to translat in E. coli, both CTG and CAG are said to be readily translatable (high frequency of use) having the advantage for expression in E. coli. CCC is also thought to have a slight edge over CCA.

Reference Example 9.

Synthesis of Prourokinase Gene Modified at 5′ end Region (Fig. 5)

The structure of a 30 bp of the 5′-end portion of the naturally occurring cDNA coding for prourokinase constructed in Reference Example 7 was altered so as to permit the prourokinase gene to be efficiently expressed in E. coli under the SD sequence of the Pseudomonas putide-derived C230 gene.

The following three single-chain DNA oligomers comprising 29, l5, and 20 nucleotides respectively were synthesized by the phosphotriester method :

```
5' CATGAGCAACGAGCTCCACCAGGTTCCGT 3'

  3' TGCAGTACTCGTTGC 5'

            3' TCGAGGTGGTCCAAGGCAGC 5'
```

Next, l µg each of the synthetic DNA oligomers was heated for 2 minutes at 95°C, phosphorylated at the 5′ end with T4 polynucleotide kinase and purified using a Sep Pak (Cl8) column (made by Waters). After drying, the purified material was dissolved in 50 µl of 20 mM Tris-HCl (pH 7.6) and l0 mM LgCl₂, and annealed by heating for 2 minutes at 95°C, cooling slowly to room temperature, and then maintaining the solution overnight at l2°C to give the following double-stranded DNA :

```
5'      CATGAGCAACGAGCTCCACCAGGTTCCGT 3'

3' TGCAGTACTCGTTGCTCGAGGTGGTCCAAGGCAGC

        AatII           SstI   BstNI   TaqI
```

On the one hand, 5 μg of DNA of plasmid pKYU22 was digested twice with restriction endonuclease BglII and AatII, and about 5.7 Kb DNA fragment was recovered by electric elution. On the other hand, 5 μg of DNA of the same plasmid pKYU22 was twice digested with restriction endonucleases PstI and BglII and about 400 bp DNA fragment was obtained by electric elution method. This fragment was again digested with restriction endonucleases TaqI and about 260 bp DNA fragment was recovered by electric elution. These two different DNA fragments were recovered and purified by phenol/chloroform extraction, and precipitation with 2 volumes of ethanol.

These two different DNA fragments and the aforesaid double-stranded synthetic DNA oligomer were ligated together using T4DNA ligase and were transformed into E. coli χI776. Then the transformants were screened by the rapid isolation method by the alkali lysis procedure, and a clone Escherichia coli χI776/pKMUI carrying plasmid pKMUI that contains a modified prourokinase gene was obtained. The clone E. coli χI776/pKMUI has been deposited with Fermentation Research Institute, Agency of Science and Technology as FERM P-8040.

Reference Example I0.

Construction of Prourokinase Directly

Expressive Plasmid (pMUT4L)

Five μg of plasmid pKMUI from Reference Example 9 was digested with I0 units of restriction endonuclease AatII and the digest was isolated after treatment with calf intestinal phosphatase (CIP). On the other hand, 5 μg of plasmid pTCMI was digested with I0 units of restriction endonuclease AatII and about 500 bp DNA fragment was isolated by the electric elution method. These two different DNA fragments were purified by repeated phenol/chloroform extraction and ethanol precipitation.

Both of these DNA fragments were joined together using T4DNA ligase and were transformed into E. coli JMI03. The transformants were screened by the rapid isolation method by the alkali lysis procedure, and a clone carrying plasmid pMUTIL in which tac promoter/operator and C230SD sequence having the normal orientation with reference to the prourokinase gene was obtained.

Said plasmid pTCMI which is a novel plasmid constructed by the present inventors contains an expression controlling region comprising tac promoter/operator, lac SD, C230SD sequences as well as the C230 structural gene, E. coli JMI03/pTCMI carrying said plasmid has been deposited with Fermentation Research Institute, Agency of Science and Technology as FERM P-7779).

In plasmid pMUTIL the modified prourokinase gene of the present invention is inserted at an appropriate site downstream of the expression controlling region comprising tac promoter/operator, lac SD and C230SD sequences.

Next, 5 μg of plasmid pKK223-3 (Literatures 22, 23 and 24) was digested with I0 units of restriction endonuclease HindIII, and the digest was treated with calf intestinal phosphatase (P.L. Biochemicals).

On the other hand, I μg of the resulting plasmid pMUTIL was digested with 4 units of restriction endonuclease DraI and the digestion fragment and I μg of 5'-phosphorylated HindIII linker (dCAAGCTTG) were ligated with T4DNA ligase. Digestion was carried out using I2 units of restriction endonuclease HindIII, and the digest was dissolved in 0.I5 M NaCl. The solution was extracted with an equal volume of phenol/chloroform and the DNA was precipitated by the addition of 2 volumes of ethanol. The precipitate was collected at I6,000 rpm and at 4°C and was dried.

The resultant pMUTIL digestion fragment and the HindIII digestion fragment of aforesaid pKK223-3 were ligated using T4 DNA ligase and were transformed into E. coli JMI03. The transformants were screened by the alkali lysis procedure and a clone, E. coli JMI03/pMUT2L containing plasmid pMUT2L was obtained.

This plasmid not only has said expression control region upstream from the prourokinase gene but also has a transcription terminator (rrnB, $T_1 T_2$) of the E. coli ribosomal gene derived from pKK223-3 downstream therefrom. Plasmid pKK223-3 is readily obtainable as it is marketed by Pharmacia P-L Biochemicals.

Five μg of plasmid pMUT2L was digested with I0 units each of restriction endonucleases SphI and TthIII I, and after extraction with phenol/chloroform DNA was precipitated with ethanol. The DNA thus recovered was blunt-ended using T4 polymerase in the presence of 0.I mM dGTP, dCTP, dATP and TTP, and was recycled by T4 DNA ligase. The DNA was transformed into E. coli JMI03 and colonies were allowed to form on an LB agar medium containing 50 μg/ml of ampicillin. The transformants were screened by the alkali lysis procedure (literature I4) and a clone, E. coli JMI03/pMUT4L was obtained.

Example I.

Introduction of Specific Base Substitution Mutation into the Codon for Amino Acid I35 Using Phage MI3 (I)

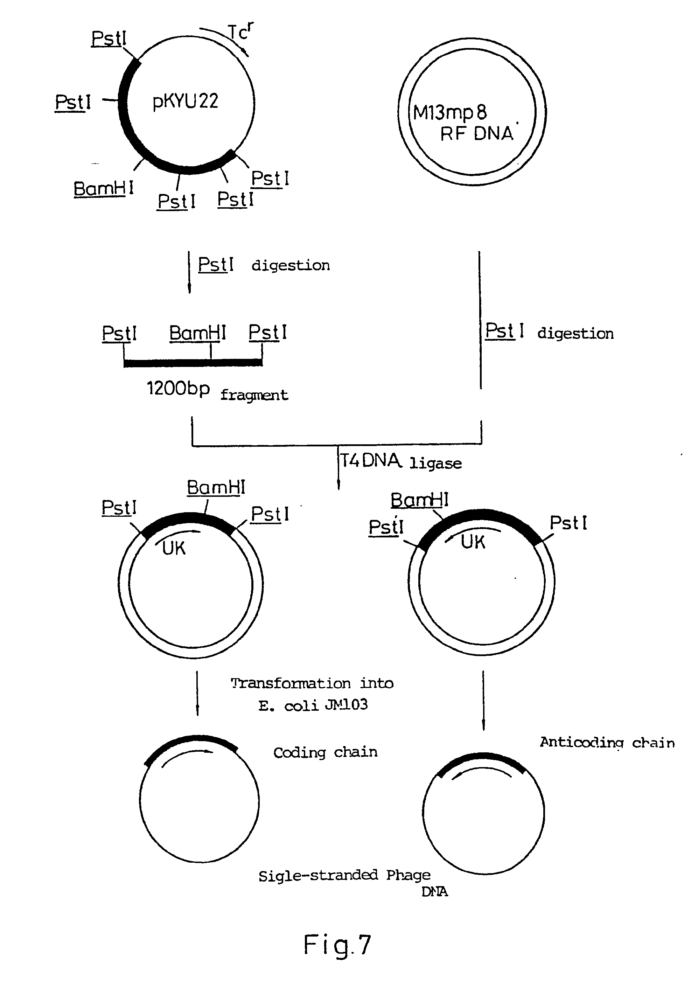

(I) Preparation of Single-stranded Template DNA (Fig. 7)

One μg each of plasmid pKYU22 and phage MI3pm8 double-stranded DNA were digested in 20 μl of a solution containing I0 mM Tris-HCl (pH 7.5), 7 mM $MgCl_2$, 7 mM β-mercaptoethanol, and 50 mM NaCl for one hour at 37°C using 5 units of PstI. The respective DNA fragments were recovered after treatment with phenol and precipitation with ethanol. A mixture of the two different DNA fragments were subjected to ligation reaction in 20 μl of a solution containing 66 mM Tris-HCl (pH 7.5), 5 mM $MgCl_2$, 5 mM DTT, and I mM ATP for I6 hours at I2°C using I00 units of T4DNA ligase. Transformation of E. coli JMI03 was carried out using the reaction mixture in accordance with the method of Messing et al (literature 25) and the transformants were plated on soft agar containing 0.02%X-gal and I mM IPTG. The plates were cultured overnight at 37°C. Single-stranded DNA was prepared from white plaques formed by the recombinant. That is, the white plaque was lifted on the tip of a toothpick, suspended in I.5 ml of 2X YT liquid medium (I.6% Bactotryptone, I% yeast extract, and 0.5% NaCl) in which E.coli JMI03 was growing, and incubated for 5 hours at 37°C. Single-stranded recombinat phage DNA was recovered from the supernatant of the liquid medium via precipitation with polyethylene glycol, treatment with phenol, and precipitation with ethanol.

Using the resultant single-stranded DNA as a template and by the dideoxy procedure in accordance with the method of Messing et al (literature 25) the base sequence was determined, and the sequence of the cloned single-stranded DNA was confirmed. As shown in Fig. 7, the coding chain and the anticoding chain were obtained.

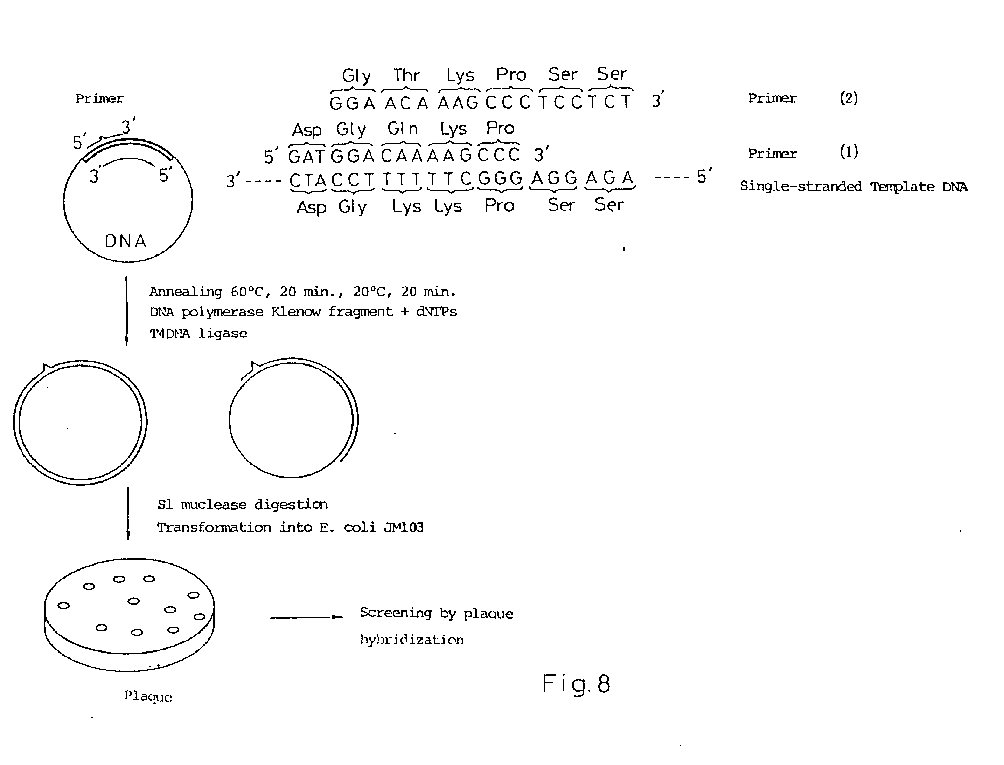

(2) Synthesis of Double-stranded DNA Using Oligonucleotide as Primer (Fig. 8)

Using the cloned anticoding chain of the resultant single-stranded DNA as a template and synthetic oligonucleatide

5' GATGGACAAAAGCCC 3'

as a primer (I), repair reaction was carried out with a DNA polymerase Klenow fragment. That is, 2 pmole of 5'-phosporylated primer was added to 0.5 pmole of the template single-stranded DNA and the mixture was incubated in I0 μl of a solution containing 7 mM Tris-HCl (pH 7.5), 0.I mM EDTA, 20 mM NaCl, and 7 mM $MgCl_2$ for 20 minutes at 60°C followed by further incubation for 20 minutes at 23°C. Next, to the reaction mixture were added respectively dATP, dGTP, dTTP, and dCTP to a final concentration of 0.5 mM to make the whole volume 20 μl to which was added 2 units of DNA polymerase Klenow fragment. The mixture was incubated for 20 minutes at 23°C and after the addition of I μl of I0 mM ATP and I unit of T4 DNA ligase the mixture was reincubated overnight at I2°C.

(3) Digestion with SI Nuclease

Digestion with SI nuclease was carried out to eliminate from the resultant double-stranded DNA the nonreacted single-stranded DNA serving as a background. That is, 2 μl of aforesaid reaction mixture was dissolved in 25 μl of a solution containing 280 mM NaCl, 4.5 mM $ZnCl_2$, and 30 mM NaOAc (pH 4 to 4.5) and after the addition of 2.3 units of SI nuclease the mixture was reacted for 30 minutes at 26°C. On addition of I0 μl of 0.25M Tris-HCl (pH 8.0) the reaction was terminated.

(4) Transformation of E. coli JMI03

Using I0 μl of aforesaid reaction mixture, the transformation of E. coli JMI03 was carried out in accordance with the method of Messing et al (literature 25).

(5) Screening of Mutants

Using as a probe the oligonucleotide (labeled with $^{32}$P) used previously as a primer, the resulting phage plaques were screened for mutant phage by plaque hybridization. That is, in accordance with the method of Benton and Davis et al (literature 26) the plaque was transferred from a soft agar medium to a nitrocellulose filter and baked in vacuo for 2 hours at 80°C. Using the $^{32}$P-labeled primer oligonucleotide as a probe, the nitrocellulose filter was hybridized overnight in a solution containing 6 x SSC and 10 x Denhardt at 23°C. The filter was then washed in 6 x SSC at 46°C and mutant phage plaques giving positive signals were autoradio-graphically isolated. This mutant phage resulted in mutant phage DNA of the double-stranded type (pml).

(6) Determination of Base Sequence of Mutant DNA

Using the mutant phage DNA as a template, the base sequence was determined via the dideoxy procedure and the occurrence of single-base substitution mutation was confirmed.

Example 2.

Introduction of Specific Base Substitution

Mutation into the Codon of Amino Acid I35 Using Phage MI3 (2)

Using the same recombinant MI3 phage single-stranded DNA (the anticoding chain of the urokinase gene has been cloned in this DNA) as that used in Example I as a template, the introduction of site specific mutation was carried out with a new oligonucleatide mutagen. In this case, the synthetic oligonucleotide,

5′ GGAACAAAGCCCTCCTCT 3′

was used as a primer (2). While this I8-base oligonucleotide is complementary to the urokinase gene in the single-stranded template DNA only one base is changed as can be seen from the change of codon AAA for lysine to codon ACA for threonine.

Using this oligonucleotide as a primer, double-stranded DNA was synthesized in vitro. That is, 2 pmole of 5′-phosphorylated primer was added to 0.5 pmole of template single-stranded DNA and the mixture was incubated in I0 µl of a solution containing 7 mM Tris-HCl (pH 7.5), 0.I mM EDTA, 20 mM NaCl, and 7 mM MgCl$_2$ for 20 minutes at 60°C. Further incubation was carried out for 20 minutes at 23°C. Next, to the reaction mixture were added respectively dATP, dGTP, dTTP, and dCTP to a final concentration of 0.5 mM to make the combined volume 20 µl to which was added 2 units of DNA polymerase Klenow fragment. The mixture was incubated for 20 minutes at 23°C, and after the addition of I µl of I0 mM ATP and I unit of T4 DNA ligase the mixture was incubated overnight at I2°C. In accordance with the method of Messin et al (literature 25), the aforesaid reaction mixture was directly transformed into E. coli JMI03. In this manner about I0,000 phage plaques per microliter of said reaction mixture were obtained.

After the transfer of the resultant plaques from a soft agar medium to a nitrocellulose filter in accordance with the method of Benton et al (literature 26), and the filter was baked in vacuo for 2 hours at 80°C. The nitrocellulose filter was hybridized with the primer oligonucleotide labeled with $^{32}$P as a probe in a solution containing 6 x SSC and I0 x Denhardt overnight at 37°C. The filter was then washed in 6 x SSC at 52°C and mutant phage plaques giving positive signals were autoradiographically isolated. From the mutant phage a double-stranded mutant phage DNA (pm2) was obtained. Using the mutant phage DNA as a template according to the dideoxy method the nucleotide sequence of the mutant DNA was determined, and the occurrence of the desired single base substitution mutation was confirmed.

Likewise, it is possible to introduce codons coding for the amino acids listed in the left-hand column below substituting for amino acid I35 which is lysine by using oligonucleotide mutagens such as those listed in the right-hand column below.

| Amino Acid 135 | Oliconucleotide Mutagen |
|----------------|--------------------------|
| Alanine | 5' GATGGAGCAAAGCCC 3' |
| Asparagine | 5' GATGGAAACAAGCCC 3' |
| Aspartic Acid | 5' GATGGAGACAAGCCC 3' |
| Glutamic Acid | 5! GATGGAGAAAGCCC 3' |
| Phenylalanine | 5' GATGGATTCAAGCCC 3' |
| Glycine | 5' GATGGAGGTAAGCCC 3' |
| Isoleucine | 5' GATGGAATCAAGCCC 3' |
| Leucine | 5' GATGGACTGAAGCCC 3' |
| Methionine | 5' GATGGAATGAAGCCC 3' |
| Serine | 5' GATGGAAGCAAGCCC 3' |
| Valine | 5' GATGGAGTAAAGCCC 3' |
| Tryptophan | 5' GATGGATGGAAGCCC 3' |
| Tyrosine | 5' GATGGATACAAGCCC 3' |
| Proline | 5' GATGGACCGAAGCCC 3' |

Note, mutation can be induced by synthesizing DNA fragment that contains codon specifying such other amino acids as glutamine or threonine in place of the codon specifying Lys-I35 while the other codons remain unchanged, and by recombining the synthetic fragment with the corresponding segment of cDNA derived from naturally occurring mRNA, instead of the above-mentioned the point mutation introduction procedure in which the aforesaid oligonucleotide primer is used.

Expression of the polypeptide of the present invention is made possible by cleaving the resultant mutant phage DNA with PstI to give a mutated insert segment, replacing this segment with the corresponding segment of the entire coding region, inserting this coding region into an expression vector suitable for E. coli, and transforming the insert into E. coli for subsequent culturing of the transformant.

## Example 3.

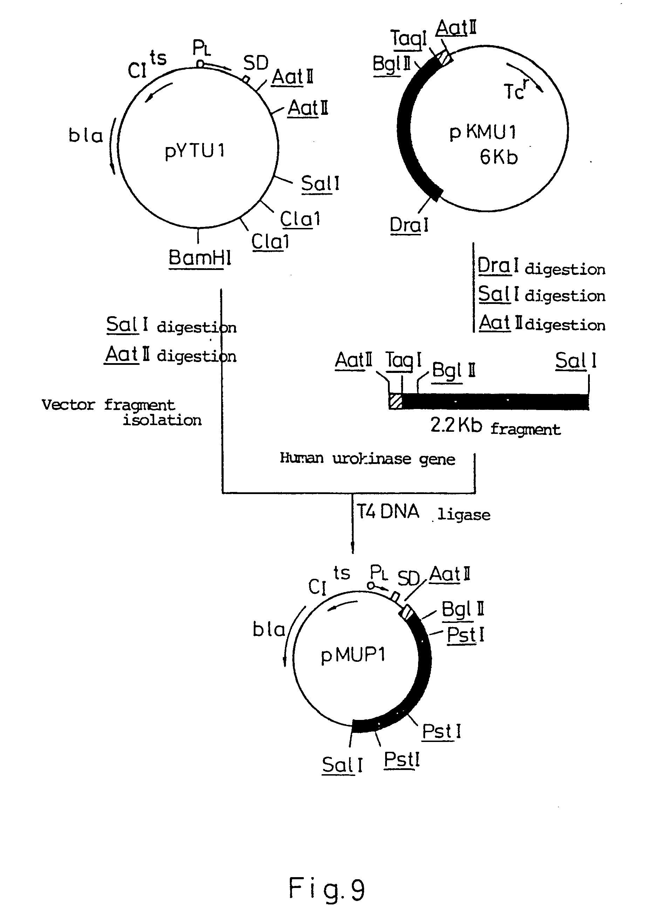

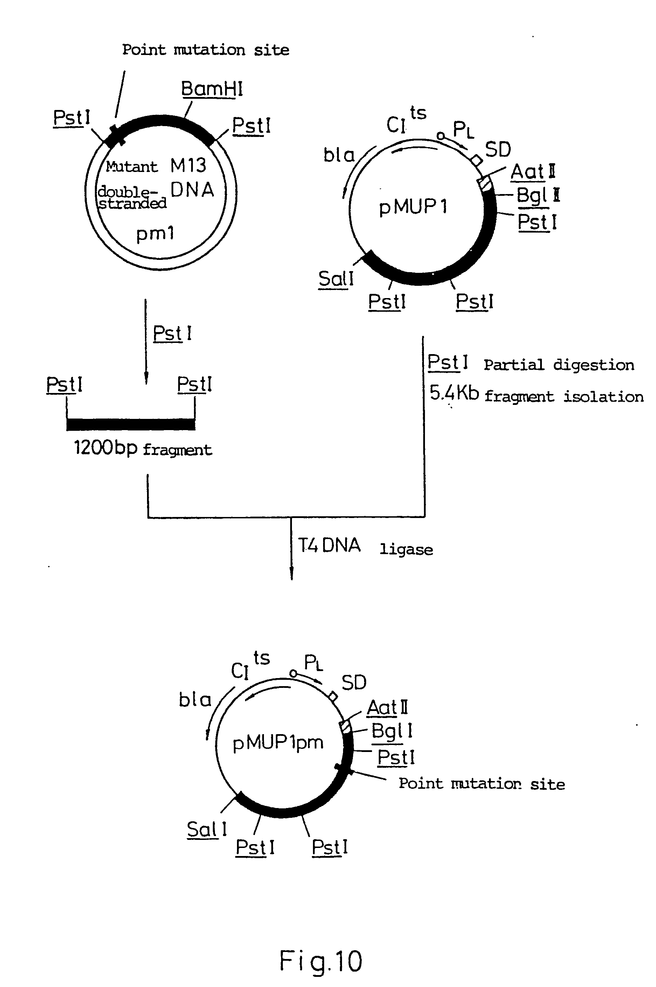

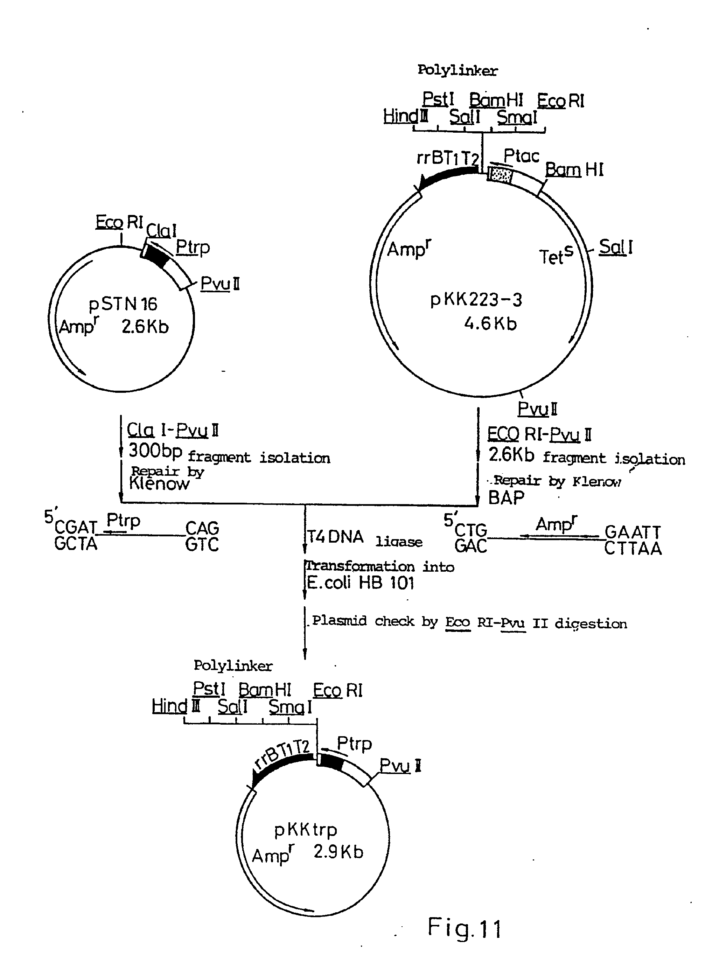

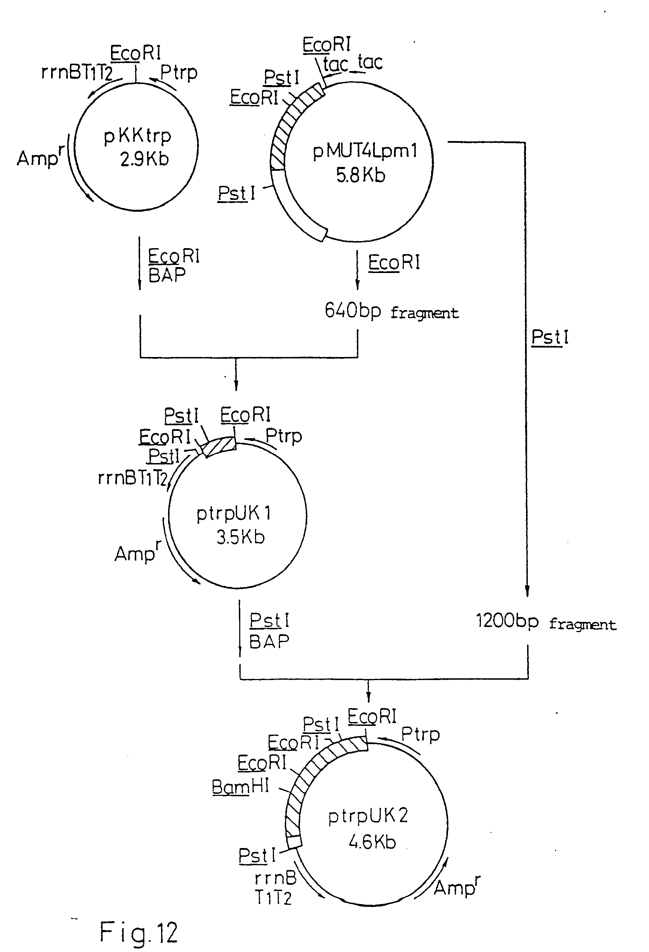

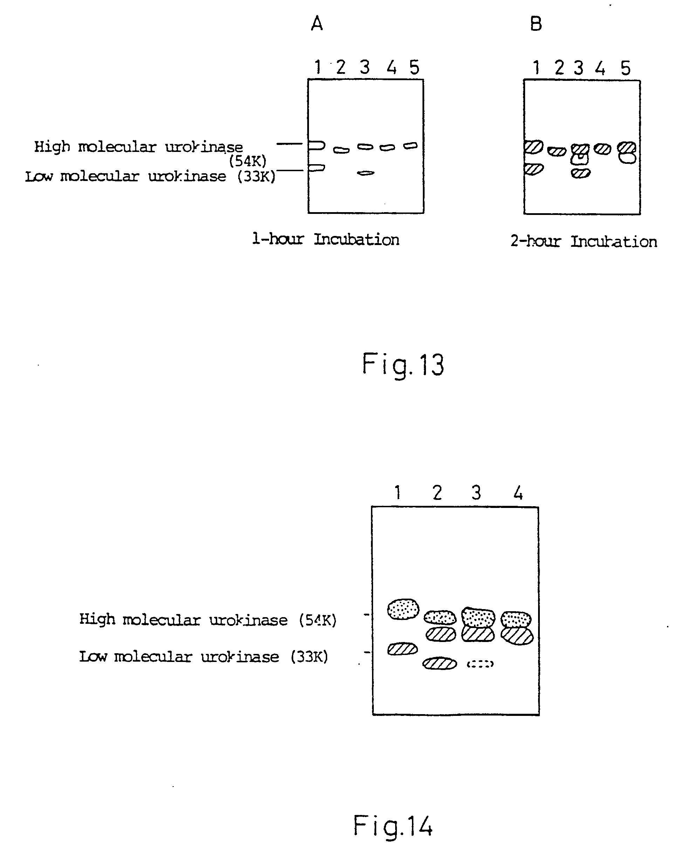

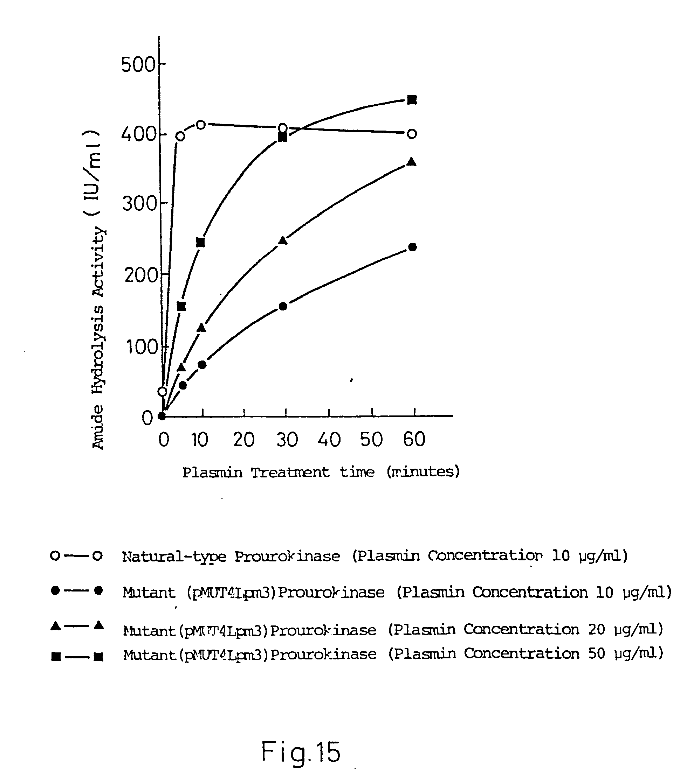

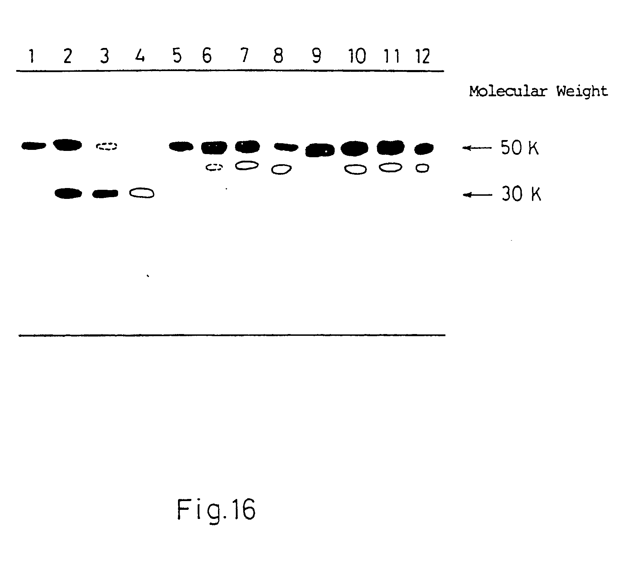

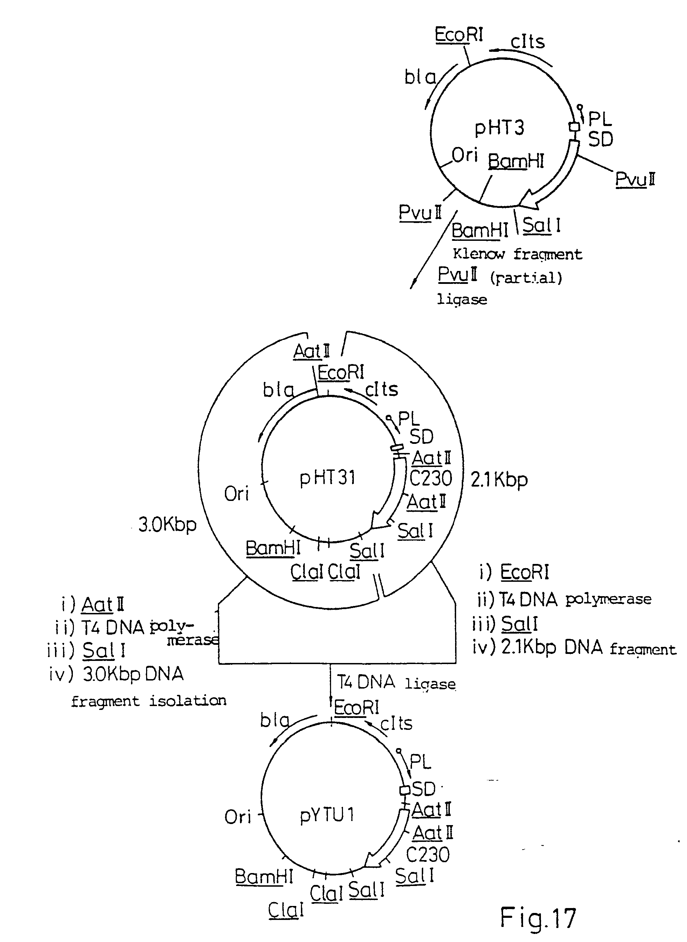

### Construction of E. coli Expression Plasmid (pMUPI) Containing the Human Prourokinase Gene (Fig. 9)

Using plasmid pYTUI (literature 27) containing $P_L$ promoter and C230-derived SD sequence as a vector, expression plasmids containing urokinase cDNA (the codon specifying a plurality of N-terminal amino acids of urokinase has been replaced as described earlier) derived from human kidney tissue were constructed. That is, I0 µg of plasmid pYTUI was digested in I00 µl of a solution containing I0 mM Tris-HCl (pH 7.4), 7 mM $MgCl_2$, and I50 mM NaCl for 2 hours at 37°C using I0 units each of Sall and AatlI and about 4.5 Kb Sall-AatlI DNA fragment was isolated.

On the other hand, I0 µg of plasmid pKMUI was digested in I00 µl of a solution containing I0 mM Tris-HCl (pH 7.4), 7 mM $MgCl_2$, and 60 mM NaCl using I0 units of Dra I for 2 hours at 37°C, and ATP was added to the digest to make the concentration 0.66 mM. After adding I µg of Sall linker DNA (5' GGTCGACC 3') and I00 units of T4 DNA ligase to the mixture the reaction was carried out overnight at I2°C. Then, DNA fragments were recovered via phenol treatment and ethanol precipitation and were digested in I00 µl of a solution containing I0 mM Tris-HCl (pH 7.4), 7 mM $MgCl_2$, and I50 mM NaCl for 2 hours at 37°C using I0 units each of AatlI and Sall, and about 2.2 Kb human urokinase DNA fragment was isolated by agarose electrophoresis. One µg each of the these two different DNA fragments were mixed and the ligation reaction was carried out overnight in 20 µl of a solution containing 66 mM Tris-HCl (pH 7.4), 7 mM $MgCl_2$, 0.66 mM ATP, and I0 mM dithiothreitol at I2°C using I00 units of T4 DNA ligase.

E. coli HBIOI was transformed using said reaction mixture and the transformants were screened by the alkali lysis procedure. There was obtained a clone carrying plasmid pMUPI from which plasmid DNA was prepared.

## Example 4.

### Construction of Plasmid pMUPlpm (Fig. I0)

Using mutant MI3 phage double-stranded DNA containing the cDNA fragment in which codon AAA specifying lysine at position I35 from the N-terminal of urokinase is mutated to codon CAA specifying glutamine, and plasmid pMUPI having the prourokinase gene downstream of $P_L$ promoter and C230 SD sequence, E. coli expression plasmid containing the mutated gene was constructed. That is, I0 µg of mutant MI3 double-stranded DNA was completely digested with PstI and a fragment about I.2 Kb in length was isolated.

On the other hand I0 µg of plasmid pMUPI was partially digested with PstI and a fragment about 5.4 Kb in length was isolated from which only a fragment about I.2 Kb in length had been eliminated.

Each of the aforementioned two different fragments were recovered by treating with phenol and precipitating with ethanol, and the precipitates were mixed. The mixture was subjected to overnight ligation reaction at I2°C using T4 DNA ligase and the reaction mixture was used to transform E. coli HBI0I. The transformants were screened by the alkali lysis procedure and a clone containing plasmid pMUPlpm was obtained. Escherichia coli χI776/pMUPlpm which carried plasmid pMUPlpm was deposited with Fermentation Research Institute, Agency of Science and Technology on January II, I985 as FERM P-8042 and was placed under International Deposition on January 22, I986 as FERM BP-969 pursuant to the provisions of the Budapest Treaty.

Plasmid pMUPlpm, which is one of the typical plasmids of the present invention, contains a region coding for the human prourokinase-like polypeptide at an appropriate site downstream from $P_L$ promoter and C230 SD sequence. In this coding region codons specifying the six N-terminal amino acids of said polypeptide have been replaced with codons which can be readily expressed in E. coli and in addition codon AAA specifying Lys at I35 position has been changed to codon CAA which specifies glutamine.

## Example 5.

### Construction of Plasmid pMUT4Lpm2 (Figures 9 and I0)

Expression plasmid pMUT4Lpm2 wherein the human prourokinase structural gene having the aforementioned point mutation had been inserted downstream of the E. coli tac promoter was constructed in the following manner :

After complete digestion of I0 µg of Mutant MI3 double-stranded DNA(pm2) with PstI, a fragment about I.2 Kb in length was isolated. On the other hand I0 µg of plasmid pMUT4L was partially digested with PstI and a fragment about 4.6 Kb in length was isolated from which only a fragment about I.2 Kb in length had been eliminated.

Each of these two fragments was recovered by treating with phenol and precipitating with ethanol, and the precipitates were mixed. The mixture was subjected to overnight ligation reaction at I2°C using T4 DNA ligase and the reaction mixture was used to transform E. coli HBI0I. The transformants were then screened by the alkali lysis procedure and a clone containing plasmid pMUT4Lpm2 was obtained. Escherichia coli χI776/pmuT4Lpm2 carrying plasmid pmuT4Lpm2 was deposited on April I8, I985 with Fermentation Research Institute, Agency of Science and Technology as FERM P-8188 and was placed under international deposition pursuant to the provisions of the Budapest Treaty on January 22, I986 as FERM BP-970.

Plasmid pMUT4Lpm2, which is also one of the typical plasmids of the present invention, contains a region coding for the human prourokinase-like polypeptide at an appropriate site downstream from tac promoter. In this coding region, codons specifying the six N-terminal amino acids of said polypeptide have been replaced with codons which can be readily expressed in E. coli, and in addition codon AAA specifying Lys at I35 position has been changed to codon ACA which specifies threonine.

## Example 6.

### Introduction of Specific Base Substitution Mutation into Codon of Amino Acid I57 Using MI3 Phage

#### (1) Preparation of Single-stranded Template DNA

One µg each of plasmid pKYU22 and phage MI3mp8 double-stranded DNA was digested in 20 µl of a solution containing I0 mM Tris-HCl (pH 7.5), 7 mM $MgCl_2$ , 7 mM β-melcaptoethanol, and 50 mM NaCl for one hour at 37°C using 5 units of PstI. The respective DNA fragments were recovered after treatment with phenol and precipitation with ethanol. A mixture of these two different DNA fragments was subjected to ligation reaction

in 20 μl of a solution containing 66 mM Tris-HCl (pH 7.5), 5 mM MgCl$_2$, 5 mM DTT, and I mM ATP for I6 hours at I2°C using T4 DNA ligase. Transformation of E. coli JMI03 was carried out using the reaction mixture in accordance with the method of Messing et all (literature 25) and the transformants were plated with soft agar containing 0.02% X-gal and I mM IPTG. The plates were incubated overnight at 37°C. Single-stranded DNA was prepared from white plaques formed by the recombinant.

Using some of the resultant single-stranded DNA as a template the base sequence was determined using the dideoxy procedure in accordance with the method of Messing et al and sequence of the cloned single-stranded DNA was confirmed.

(2) Introduction of Specific Base Substitution Mutation

Using the resulting recombinant MI3 phage single-stranded DNA (an anticoding chain of the urokinase gene has been cloned) as a template, introduction of site-specific mutations was carried with a another oligonucleotide mutagen. In this case, the following synthetic oligonucleotide :

5' GGCCCCGCGATAAGATTA 3'

was used as a primer. Although this I8-base oligonucleotide is complementary to the urokinase gene in the single-stranded template DNA, two bases have undergone changes wherein codon TTT which specicies phenylalanine has changed to codon GAT specifying aspartic acid.

Using the aforesaid oligonucleotide as a primer, double-stranded DNA was synthesized in vitro. That is, 2 pmole of 5'-phosphorylated primer was added to 0.5 pmole of the template single-stranded DNA and the mixture was incubated in I0 μl of a solution containing 7 mM Tris-HCl (pH 7.5), 0.I mM EDTA, 20 mM NaCl, and 7mM MgCl$_2$ for 20 minutes at 60°C followed by further incubation for 20 minutes at 23°C. Next, to the reaction mixture was added 0.5 mM each of dATP, dGTP, dTTP and dCTP to a combined volume of 20 μl to which was added 2 units of DNA polymerase Klenow fragment. The mixture was incubated for 20 minutes at 23°C, and after the addition of I μl of I0 mM ATP and I unit of T4 DNA ligase the mixture was incubated overnight at I2°C. In accordance with the method of Messing et al the aforesaid reaction mixture was directly transformed into E. coli JMI03. About I0,000 phage plaques per microliter of said reaction mixture were thus obtained.

After the transfer of the resulting plaques from a soft agar medium to a nitrocellulose filter in accordance with the method of Benton and Davis et al (Literature 26), the filter was baked in vacuo for 2 hours at 80°C. The nitrocellulose filter was hybrilized with the primer oligonucleotide labeled with $^{32}$P as a probe in a mixture of 6 x SSC and I0 x Denhardt overnight at 37°C. The filter was then washed in 6 x SSC at 52°C and mutant phage plaques giving positive signals were autoradiographically isolated. From the mutant phage, mutant phage DNA of the double-stranded type (pm3) was obtained. Using the mutant phage DNA as a template according to the dideoxy method, the nucleotide sequence of the mutant DNA was determined, and the occurrence of the desired single base substitution mutation was confirmed.

In addition, it is possible to replace codon TTT specifying phenylalanine at I57 position with the Glu-specifying codon GAA using the following oligonucleotide :

5' GGCCCCGCGAAAAGATTA 3'

as a mutagen.

Example 7.

Construction of Plasmid pMUT4Lpm3

Expression plasmid pMUT4Lpm3 wherein the human urokinase structural gene having the aforementioned point mutation had been inserted downstream of E. coli tac promoter was constructed in the following manner:

After complete digestion of I0 μg of mutant MI3 double-stranded DNA (pm3) with PstI, a fragment about I.2 Kb in length was isolated. On the other hand, I0 μg of plasmid pMUT4L was partially digested with PstI and a fragment about 4.6 Kb in length was isolated from which a fragment about I.2 Kb in length had been eliminated.

Each of the above two fragments was recovered by treating with phenol and precipitating with ethanol and the precipitates were mixed. The mixture was subjected to ligation reaction overnight at I2°C using T4 DNA ligase and the reaction mixture was used to transform E. coli HBI0I. The transformants were then screened by the alkali lysis procedure and a clone containing plasmid pMUT4Lpm3 was obtained. Escherichia coli χI776/pMUT4Lpm3 carrying plasmid pMUT4Lpm3 was deposited on July II, I985 with Fermentation Research Institute, Agency of Science and Technology as FERM P-834I and was placed under international deposition on January 22, I986 as FERM BP-97I pursuant to the provisions of the Budapest Treaty.

Plasmid pMUT4Lpm3, which is one of the typical plasmids of the present invention, contains a region coding for the human prourokinase-like polypeptide at an appropriate site downstream from tac promoter. In this coding region, codons specifying the six N-terminal amino acids of said polypeptide have been replaced with codons which can be readily expressed in E. coli and, in addition, codon TTT which specifies phenylalanine at l57 position has been changed to codon GAT which specifies aspartic acid.

## Example 8.

### Construction of Plasmid pKKtrp (Fig. II)

Five µg of plasmid pKK223-3 was reacted with 30 units of EcoRI and 20 units of PvuII in l00 µl of buffer (l0 mM Tris-Cl, pH 7.5, l0 mM $MgCl_2$, 50 mM NaCl, and l mM DTT) for one hour at 37°C. The reaction mixture was subjected to 0.7% agarose electrophoresis and a DNA fragment about 2,600 bp in length containing the ampicillin resistant gene, the replication initiation region and $rrnBT_1T_2$ transcription termination region was conventionally recovered.

The above fragment was reacted with one unit of Klenow fragment in 25 µl of buffer (50 mM Tris-Cl, pH 7.2, l0 mM $MgSO_4$, 0.l mM DTT, and 80 µM dNTPs) for one hour at 25°C and after the addition of one unit of alkaliphosphatase the mixture was allowed to undergo reaction for half an hour at 68°C. The reaction product was precipitated with ethanol after phenol treatment and the precipitate was dissolved in 20 µl of TE buffer (l0 mM Tris-HCl, pH 8.0 and l mM EDTA). The resulting DNA fragment was designated as [A].

On the other hand, l0 µg of plasmid pSTNI6 and 20 units each of ClaI and PvuII were reacted in l00 µl of a buffer (l0 mM Tris-Cl, pH 7,5, l0 mM $MgCl_2$, 50 mM NaCl, and l mM DTT) for one hour at 37°C, and the reaction mixture was then subjected to 0.7% agarose electrophoresis. There was conventionally recovered a fragment about 300 bp in length containing a promoter, an operator, and a ribosome binding site of tryptophan operon.

The above fragment and one unit of Klenow fragment were reacted in 25 µl of buffer (50 mM Tris-HCl, pH 7.2, l0 mM $MgSO_4$, 0.l mM DTT, and 80 µM dNTPs) for one hour at 25°C, and after phenol treatment and ethanol precipitation of the reaction mixture, the precipitate was dissolved in 20 µl of TE buffer. The DNA fragment thus obtained was designated as [B].

Next, 0,5 µg each of DNA fragments [A] and [B] were reacted with 2.5 units of T4DNA ligase in 20 µl of buffer (66 mM Tris-HCl, pH 7.6, 6.6 mM $MgCl_2$, l0 mM DTT, and l mM ATP) for l5 hours at l5°C, and the reaction product was used to transform E. coli HBl0l. The ampicillin resistant transformants were screened for colonies carrying plasmid pKKtrp and the desired plasmid was conventionally isolated.

## Example 9.

### Construction of Plasmid ptrpUK2 (Fig. l2)

By using plasmids pKKtrp and pMUT4L as the starting materials, plasmid ptrpUK2 in which the human prourokinase gene had been inserted downstream from tryptophan promoter was constructed.

That is, l µg of plasmid pKKtrp and 5 units of EcoRI were reacted in 50 µl of buffer (l0 mM Tris-HCl, pH 7.5, l0 mM $MgCl_2$, 50 mM NaCl, and l mM DTT) for 2 hours at 37°C. Then, after the addition of 2 units of alkaliphosphatase, a further reaction was carried out for 30 minutes at 65°C, and following phenol treatment and ethanol precipitation the desired DNA fragment (C) was recovered.

On the other hand, l0 µg of plasmid pMUT4L and 20 units of EcoRI were reacted in l00 µl of the same buffer as above for 2 hours at 37°C. After the termination of the reaction, a 640 bp DNA fragment containing a N-terminal region of the human urokinase gene was isolated by l.2% agarose electrophoresis. This DNA fragment was designated as [D].

DNA fragments [C] and [D] were reacted with 2.5 units of T4 DNA ligase in l0 µl of buffer (66 mM Tris-HCl, pH 7.6, 6.6 mM $MgCl_2$, l0 mM DTT, and l mM ATP) for l5 hours at l2°C and the reaction product was used to transform E. coli HBl0l. The ampicillin resistant transformants were screened for colonies carrying plasmid ptrpUKl and the desired plasmid was conventionally isolated.

Next, l µg of plasmid ptrpUKl and 5 units of PstI were reacted in 50 µl of buffer (l0 mM Tris-HCl, pH 7.5, l0 mM $MgCl_2$, 50 mM NaCl, and l mM DTT) for 2 hours at 37°C. Then, after the addition of 2 units of alkaliphosphatase, a further reaction was carried out for 30 minutes at 65°C, and following phenol treatment and ethanol precipitation, the desired DNA fragment [E] was recovered.

On the other hand, l0 µg of plasmid pMUT4L and 20 units of PstI were reacted in l00 µl of the same buffer as above for 2 hours at 37°C. After the termination of the reaction, a l.2 Kb DNA fragment [F] containing a C-terminal region of the human urokinase gene was isolated by 0.7% agarose electrophoresis.

DNA fragments [E] and [F] were reacted with 2.5 units of T4 DNA ligase in l0 µl of buffer (66 mM Tris-HCl, pH 7.6, 6.6 mM $MgCl_2$, l0 mM DTT, and l mM ATP) for l5 hours at l2°C, and the reaction mixture was used to

transform E. coli HBI0I. The ampicillin resistant transformants were screened for colonies carrying plasmid ptrpUK2 and the desired plasmid was conventionally isolated.

## Example I0.

### Construction of Plasmid ptrpUK2 (II35)

Double-stranded mutant phage MI3RF (II35), into which a DNA fragment whose codon AAA specifying lysine at I35 position having been mutationally changed to codon ATA specifying isoleucine had been inserted, was obtained by the same method as that of Example 1 except that the following synthetic oligonucleotide :

5′ CAGATGGAATAAAGCC 3′

was used as a primer.

Ten μg of phage MI3RF (II35) was completely digested with PstI and a DNA fragment about I.2 Kb in length was isolated. On the other hand, I μg of plasmid ptrpUK2 was completely digested with PstI and a DNA fragment about 3.4 Kb in length intended as a vector was isolated. These two DNA fragments were recovered by treating with phenol and precipitating with ethanol, and the precipitates were mixed. The mixture was reacted overnight using T4 DNA ligase and the reaction mixture was used to transform E. coli HBI0I. The ampicillin resistant colonies were screened and a clone containing plasmid ptrpUK2 (II35) was obtained. All the conditions used in this procedure were the same as those described in Example 4.

Escherichia coli W3II0/ptrpUK2 (II35) containing this plasmid was deposited on December 28, I985 with Deutsche Sammelung von Mikroorganismen (Grisebachstrasse 8 D-3400 Goetingen) as DSM-3622 pursuant to the provisions of the Budapest Treaty.

## Example II.

### Construction of Plasmid ptrpUK (EI35)

A double-stranded mutant phage MI3RF (EI35), into which a DNA fragment whose codon AAA specifying lysine at I35 position having been mutationally changed to codon GAA specifying glutamic acid had been inserted, was obtained by the same method as that of Example I except that the following synthetic nucleotide :

5′ GATGGAGAAAAGCCT 3′

was used as a primer.

Next, plasmid ptrpUK2 (EI35) was obtained from phage MI3RF (I35) and plasmid strpUK2 by the same method as that described in Example I0.

Escherichia coli W3II0/ptrpUK2 (EI35) containing this plasmid was deposited on December 28, I985 with Deutsche Sammelung von Mikroorganismen as DSM-362I.

## Example I2.

### Construction of Plasmid ptrpUK2 (QI35DI57)

A double-stranded mutant phage in which a DNA Fragment whose codon AAA specifying lysine at I35 position having been mutationally changed to codon CAA specifying glutamine had been inserted was obtained by the same method as that of Example I except that the following synthetic nucleotide :

5′ GATGGACAAAAGCCC 3′

was used as a primer.

Next, single-stranded phage DNA was obtained from the above phage by the same method as that described in Example I, and double-stranded mutant phage MI3RF (QI35DI57), into which a DNA fragment whose codon AAA specifying lysine at I35 position having been mutationally changed to codon CAA specifying glutamine and codon TTT specifying phenylalanine at I57 position likewise changed to codon GAT specifying aspartic acid had been inserted, was obtained in a similar manner as the above except that the following synthetic oligonucleotide :

5′ GGCCCCGCGATAAGATTA 3′

was used as a primer thereby changing codon TTT specifying phenylalanine at the I57 position to codon GAT specifying aspartic acid.

Plasmid trpUK2 (QI35DI57) was obtained from the MI3RF (QI35DI57) and plasmid ptrpUK2 by the same method as that described in Example I0.

Escherichia coli containing W3II0/p trpUK2 (QI35DI57) containing the above plasmid was deposited on December 28, 1985 with Deutsche Sammelung von Mikro-organismen as DSM-3623.

Example l3.

### Construction of Plasmid trpUK2 (El35Dl57)

Double-stranded mutant phage MI3RF (El35Dl57), into which a DNA fragment whose codon AA specifying lysine at the l35 position having been mutationally changed to codon GAA specifying glutamic acid and codon TTT specifying phenylalanine at the l57 position changed to codon GAT specifying aspartic acid had been inserted, was obtained by the same method as that of Example l2 except that the following synthetic nucleotide :

5′ GATGGAGAAAAGCCCT 3′

was used to mutationally change codon AAA specifying lysine at the l35 position to codon GAA specifying glutamic acid.

Next, plasmid trpUK2 (El35Dl57) was obtained from MI3RF (El35Dl57) and trpUK2 by the same method as that described in Example l0.

Escherichia coli W3ll0/ptrpUK2 (El35Dl57) containing this plasmid was deposited on December 28, 1985 with Deutsche Sammelung von Mikroorganismen as DSM-3624.

Example l4.

### Expression of Prourokinase-like Polypeptide (l)

Plasmids pMUPl and pMUPlpm obtained respectively in Examples 3 and 4 were conventionally transformed into E. coli W3ll0 and the resulting transformants were cultured in 50 ml of L-broth at 30°C. The temperature of the culture broth was increased to 42°C when the absorbance at 600 nm had reached about 0.3 and further incubation was carried out for 3 hours to express the urokinase gene.

Example l5.

### Extraction of Gene Product from E. coli and Its Properties (l)

The cells of E. coli W3ll0 were harvested from 5 ml of the liquid medium described above and after suspending the cells in 0.5 ml of a solution containing 7.5 M guanidine hydrochloride and 0.05 M Tris-HCl (pH 7.5), the suspension was allowed to stand for 90 minutes at room temperature. The suspension was then centrifuged for l0 minutes at l,000 rpm, and after diluting the supernatant in 5 ml of a solution containing l M guanidine hydrochloride, 0.05 M Tris-HCl (pH 7.5), 2 mM reductive glutathione, and 0.2 mM oxidative glutathione, the mixture was incubated overnight at room temperature. The mixture was then dialyzed for 4 hours at 4°C against l00 times the volume of a solution containing l0 mM Tris-HCl (pH 7.4) and 0.4 M NaCl and further dialysis was carried out for 2 hours against l00 times the volume of a solution containing l0 mM Tris-HCl (pH 7.4) and 0,l M NaCl.

Trypsin was added to part of the resultant crude extract to a final concentration of 30 µg/ml and the mixture was incubated for one hour at 37°C. A mixture containing water in place of trypsin was likewise incubated as a control.

The above mixtures were subjected to electrophoresis in a gel containing l2.5% polyacrylamide and 0.l% SDS in accordance with the method of Laemmli et al (literatire 30), and the gel was immersed in 2.5% Triton X-l00 and was incubated for one hour at room temperature. The gel was then layered over a fibrin agar plate and was incubated for one or two hours at 37°C. The molecular weight was subsequently estimated on the basis of the location of the dissolved zone on the fibrin agar plate. A standard sample of human urine-derived urokinase was electrophoretically migrated for comparison.

In Fig. l3, Lane l is an electrophoresis pattern of the standard urokinase (l unit), Lane 2 is that of the crude extract from E. coli transformed by plasmid pMUPl, Lane 3 is that of the trypsin-treated crude extract from E. coli transformed by plasmid pMUPl, Lane 4 is that of the crude extract from E. coli transformed by plasmic pMUPlpm, and Lane 5 is that of the trypsin-treated extract from E. coli transformed by plasmid pMUPlpm.

As is evident from the figure, although the gene product derived from plasmid pMUPl has a molecular weight of about 50,000, a part of the product converted to a lower molecular weight species having a molecular weight of about 30,000 on trypsin treatment. On the other hand, the gene product derived from plasmid pMUPlpm also had a molecular weight of about 50,000, but did not convert to a lower molecular weight material on trypsin treatment. The molecular weights of these products being slightly less than those of high and low molecular urokinases derived from human urine, e.g., 50,000 and 30,000 vs 54,000 and 33,000, is presumably due to a failure of the addition of glycoside chains within E. coli cells.

## Example I6.

### Expression of Prourokinase-like Polypeptide (2)

Plasmid pMUT4Lpm2 obtained in Example 5 was conventionally transformed into E. coli JMI03 and the resulting transformant was cultured in 5 ml of L-broth at 37°C. Expression was induced by adding IPTG (isopropyl-$\beta$-D-thiogalactopyranoside) to a final concentration of I mM when the absorbance at 550 nm reached about 0.5, and further culturing was carried out for 3 hours at 37°C to express the urokinase gene.

## Example I7.

### Extraction of Gene Product from E. coli and Its Properties (2)

The crude extract was prepared after treating 5 ml of aforesaid liquid medium by the same method as that described in Example I5, and part of the crude extract was treated with trypsin.

These preparations were subjected to electrophoresis in a gel containing I0% polyacrylamide and 0.I% SDS in accordance with the method of Laemmli et al (literature 30), and the gel was immersed in 2.5% Triton® X-I00, and was incubated for one hour at room temperature. The gel was layered over a fibrin agar plate, and was incubated for about 2 hours at 37°C. The molecular weight was subsequently estimated on the basis of the location of the dissolved zone on the fibrin agar plate. A standard sample of human urine-derived urokinase and the trypsin-treated preparation obtained in.Example I5 were also electrophoretically migrated for comparison.

The result is shown in Fig. I4. In this figure Lane I is an electrophoresis pattern of the standard urokinase (I unit), Lane 2 is that of the trypsintreated crude extract from E. coli transformed by plasmid pMUPI, Lane 3 is that of the trypsin-treated crude extract from E. coli transformed by plasmid pMUT4Lpm2, and Lane 4 is that of the trypsin-treated crude extract from E. coli transformed by plasmid pMUPIpm.

As is evident from the figure, the gene product derived from plasmid pMUT4Lpm2 is converted with difficulty to a low-molecular weight component as in the case of the gene product derived from plasmid pMUPIpm. The molecular weights of these product being slightly less than those of high and low molecular urokinases derived from human urine, e.g., 50,000 and 30,000 vs 54,000 and 33,000, is presumably due to failure of the addition of glycoside chains within the E. coli cells.

## Example I8.

### Expression of Prourokinase-like Polypeptide (3)

Plasmid pMUT4Lpm3 obtained in Example 7 was conventionally transformed into E. coli JMI03, and the resulting transformant was incubated in 5 ml of L-broth at 37°C. Expression was induced by adding IPTG (isopropyl-$\beta$-D-thiogalactopyranoside) to a final concentration of I mM when the absorbance at 550 nm reached about 0.5, and further culturing was carried out for 3 hours at 37°C to express the urokinase gene.

## Example I9.

### Extraction and Purification of Gene Product from E. coli (3)

The cells of E. coli JMI03 were harvested from 5 ml of aforesaid liquid medium, and after suspending the cells in 0.5 ml of a solution containing 7.5 M guanadine hydrochloride and 0.05 M Tris-HCl (pH 7.5) the suspension was allowed to stand for 90 minutes at room temperature. The suspension was then centrifuged for I0 minutes at I,000 rpm, and after diluting the supernatant in 5 ml of a solution containing I M guanadine hydrochloride, 0.05 M Tris-HCl (pH 7.5), 2 mM reductive glutathione, and 0.2 mM oxidative glutathione, the mixture was incubated overnight at room temperature. The mixture was then dialyzed for 4 hours at 4°C against I00 times the volume of a solution containing I0 mM Tris-HCl (pH 7.4) and 0.4 M NaCl, and further dialysis was carried out against I00 times the volume of a solution containing I0 mM Tris-HCl (pH 7.4) and 0.I M NaCl for two hours.

The mutant (pm3) urokinase was purified by subjecting the resulting crude extract to affinity chromatography using a Sepharose® column to which the antiurokinase monoclonal antibody has been bonded.

## Example 20.

### Expression of Prourokinase-like Polypeptide (4)

Plasmides ptrpUK2 (QI35DI57) and ptrpUK2 (EI35DI57) obtained respectively in Examples I2 and I3 were conventionally transformed into E. coli W3II0, and the resulting transformants were incubated in 5 ml of L-broth at 37°C. Expression was induced by adding IAA (indoleacetic acid) to a final concentration of 50 μg/ml. when the absorbancy at 550 nm reached about 0.5, and further culturing was carried out for 3 hours at 37°C to express the urokinase gene.

## Example 2I.

### Extraction and Purification of Gene Product from E. coli and Its Properties (4)

Of the aforesaid liquid media, 5 ml of the medium derived from plasmid ptrpUK2 (QI35DI57) was extracted and purified by the same method as that of Example I9 to give the mutant prourokinase-like polypeptide (QI35DI57). A part of the polypeptide was treated with trypsin in the following manner.

After the determination of the enzyme activities following the synthetic substrate procedure, samples were diluted with a solution containing 50 mM Tris-HCl buffer (pH 7.4), 0.I5 M sodium chloride, and 0.I% Triton® X-I00 to a final concentration of I00 IU/ml. To 50 μl of this solutions were added 3 μl aliquots of aqueous trypsin solution respectively made up to final concentrations of I mg/ml, 2 mg/ml, and 3 mg/ml, and the mixtures were incubated for 60 minutes at 37°C. The reaction was then terminated on addition of 5 μl of soybean trypsin inhibitor. Samples containing water instead of trypsin were likewise treated as controls.

The samples were then subjected to electrophoresis in a I0% - 26% polyacrylamide continuous concentration gradient gel containing 0.I% SDS in accordance with the method of Laemmli et al (literature 30), and the gell was immersed in 2.5% Triton® X-I00, and was incubated for about one hour at room temperature. The gel was layered over a fibrin agar plate and was incubated for 2 hours at 37°C. The molecular weight was estimated on the basis of the location of the dissolved zones on the fibrin agar plate. The natural-type prourokinase-like polypeptide and the mutant prourokinase-like polypeptide (QI35) obtained by extraction and purification of the liquid medium obtained in Example I4 according to the same procedure as that described in Example I9 were also treated in the same manner. The result is shown in Fig. I6. In this figure, Lanes I, 2, 3, and 4 are electrophoresis patterns of the pMUPI-derived natural- type prourokinase-like polypeptide treated after the addition of aqueous trypsin solutions respectively made up to final concentrations of 0, I, 2, and 3 mg/ml. Lanes 5, 6, 7, and 8 are those of the pMUPIpm-derived mutant prourokinase-like polypeptides (QI35) likewise treated with trypsin. Lanes 9, I0, II, and I2 are those of the ptrpUK2 derived mutant prourokinase-like polypeptide (QI35DI57) likewise treated with trypsin. As is evident from the figure, although the molecular weight of the pMUPI-derived natural-type prourokinase was about 50,000, dissociation by trypsin reduced it to 30,000. On the other hand, no conversion to lower molecular weight species took place on trypsin treatment in the case of the polypeptides derived respectively from plasmids pMUPIpm and ptrpUK2 (QI35DI57).

## Reference Example II.

### Construction of Plasmid pYTUI

Plasmid pHT3 was conventionally obtained from Escherichia coli HBI0I/pHT3 deposited with Fermentaticn Research Institute, Agency of Science and Technology as FERMP-7776).

Five mg of plasmid pHT3 and I5 units of BamHI were reacted in 20 μl of buffer (I0 mM Tris-HCl, pH 8.0, 7 mM MgCl₂, I00 mM NaCl, and 2 mM β-mercaptoethanol) for 3 hours, and after ethanol precipitation, the precipitate was reacted with I unit of Klenow fragment in 20 μl of buffer (50 mM Tris-HCl, pH 7.2, I0 mM MgCl₂, 0.I mM DTT, and 80 μM dNTP) for half an hour at 22°C. After phenol treatment and ethanol precipitation, the precipitate was reacted with 20 units of PvuII in 20 μl of buffer (I0 mM Tris-HCl, pH 7.5, 7 mM MgCl₂, 60 mM NaCl, and 7 mM β-mercaptoethanol) for 3 hours at 37°C. Then, after ethanol precipitation, the precipitate was reacted with 2.8 units of ligase in 20 μl of buffer (66 mM Tris-HCl, pH 7.6, 6.6 mM MgCl₂, I0 mM DTT, and I mM ATP) for 20 hours at I5°C. The reaction product was used to transform E. coli HBI0I.

A strain carrying plasmid pHT3I was isolated from among the transformants and then plasmid pHT3I was

conventionally obtained.

Next, IO µg of plasmid pHT3I was reacted with 20 units of AatII in 20 µl of buffer (IO mM Tris-HCl, pH 7.5, 7 mM MgCl$_2$ , 60 mM NaCl, and 7 mM β-mercaptoethanol) for 3 hours at 37°C, and after ethanol precipitation the precipitate was reacted with 2.8 units of T4 DNA polymerase in 20 µl of buffer (67 mM Tris-HCl, pH 8.8, 6.7 mM MgCl$_2$ , IO mM β-mercaptoethanol, 6.7 mM EDTA, I6.6 mM (NH$_4$)$_2$SO$_4$ , and 330 µM dCTP) for I5 minutes at 37°C. After phenol treatment and ethanol precipitation the precipitate was then reacted with 20 units of SalI in 20 µl of buffer (IO mM Tris-HCl, pH 7.5, 7 mM MgCl$_2$ , I50 mM NaCl, 0.2 mM EDTA, and 7 mM β-mercaptoethanol) for 3 hours at 37°C. The DNA reaction mixture was subject to I.2% agarose gel electrophoresis and the DNA fragment about 3,000 bp in length containing the ampicillin resistant gene and the replication initiation region was conventionally recovered. This DNA fragment was designated as DNA fragment [A].

On the other hand, IO µg of plasmid pHT3I was reacted with 30 units of EcoRI in 20 µl of buffer (I00 mM Tris-HCl, pH 7.5, 7 mM MgCl$_2$ , and M β-mercaptoethanol) for 3 hours at 37°C, and after ethanol precipitation the precipitate was reacted with 2.8 units of T4 DNA polymerase in 20 µl of buffer (67 mM Tris-HCl, pH 8.8, 6.7 mM MgCl$_2$ , IO mM β-mercaptoethanol, 6.7 mM EDTA, I6.6 mM (NH$_4$)$_2$SO$_4$ , and 330 µM dCTP) for I5 minutes at 37°C. After phenol treatment and ethanol precipitation the precipitate was then reacted with 20 units of SalI in 20 µl of buffer (IO/mM Tris-HCl, pH 7.5, 7 mM MgCl$_2$ , I50 mM NaCl, 0.2 mM EDTA, and 7 mM β-mercaptoethanol) for 3 hours at 37°C. The DNA reaction mixture was subjected to I.2% agarose gel electrophoresis and the DNA fragment about 2,I00 bp in length containing the clts and P$_L$ promoter-operator was recovered. This DNA fragment was designated as DNA fragment [B]. 0.5 µg each of DNA fragments [A] and [B] was reacted with 2.5 units of T4 DNA ligase in 20 µl of buffer (66 mM Tris-HCl, pH 7.6, 6.6 mM MgCl$_2$ , IO mM DTT, and I mM ATP) for I5 hours at I5°C, and the reaction product was used to transform E. coli HBI0I. The ampicillin resistant transformants were screened for colonies carrying plasmid pYTUI shown in Fig.II and the plasmid was conventionally isolated.

Literature

(I)   Samama, M,, Castel, M,, Matsuo, O., Hoylaert's, M. and Lijnen, H.R. (I982) Thromb. Haemostas. 47, 36-40.

(2)   Wun T., Ossowski L. and Reich E. (I982) J. Biol. Chem., 257, 7262-7268.

(3)   Husain S.S,, Gurewich V. and Lipinski B. (I98I) Thromb. Haemostas 46, II.

(4)   Gurewich, V., Pannell, R., Louie, S., Kelley, P., Suddith, R.L. and Greenlee, R. (I984) J. Clin. Invest. 73 I73I- I739.

(5)   Nolan C., Hall L.S., Ballow G.H. and Tribby I.I.E. (I977) Biochim. Biophys. Acta 496 384-400.

(6)   Barlow G.H., Eur. Urokinase Symp. (I98I) pI to 5.

(7)   Suyama T. (I977) Medical Postgraduate I5, 547-55I.

(8)   Steffens, G.J., Günzler, W.A. Ötting, F., Frankus, E. and Flohé, L. (I982) Hoppe-Seyler's Z. Physiol. Chem. 363, I043-I058.

(9)   Günzler, W.A., Steffens, G.J., Ötting, F., Kim, S.A., Frankus, E. and Flohé, L. (I982) Hoppe-Seyler's Physiol. Chem. 363, II55-II65.

(I0)  Japanese Patent Application No. 59-97I06.

(II)  Chirgwin, J.M., Przybyla, A.E., MacDonald, R.J. and Rutter, W.J. (I979) Biochemistry I8, 5294-5299.

(I2)  Aviv, H. and Leder, P. (I972) Proc. Natl, Acad. Sci. USA 69, I408-I4I2.

(I3)  Maniatis, T., Fritsch, E.F. and Sambrook, J. (I982) In : Molecular Cloning, Cold Spring Harbor Laboratory, Cold Spring Harbor, N.Y., pp.254.

(I4)  Birnboim, H.C. and Doly, J. (I979) Nucl. Acids Res. 7, I5I3-I523.

(I5)  Grunstein M. and Hogness D.S., (I975) Proc. Natl, Acad, Sci., USA, 72, 396I-3965.

(I6)  A.M. Maxam, and W. Gilbert (I980) Methods Enzymol. 65, 499-560.

(I7)  Messing, J., Grec, R. and Seeburg, P.H. (I98I) Nucl. Acids Res. 9, 309-32I.

(I8)  Agarwall, K.L., Brunstedt, J. and Noyes, B.E. (I98I) J, Biol. Chem. 256, I023-I028.

(I9)  Stewart A.G., Richards H., Roberts S., Marwick J., Edwards K., I. Bell, J. Smith and R. Derbyshire (I983) Nuc. Acids Res. II, 6597-6609.

(20)  Japanese Unexamined Patent Publication No. 59-5I300.

(2I)  Ikemura, T. and Ozeki, H. (I983) Cold Spring Harbor Symp. Quart. Biol. 47, I087.

(22)  Brosius J., Ullrich A., Raker M.A., Gray A., Dull T.J., Gutell R.R. and Noller H.F., (I98I) plasmid 6, II2-II8.

(23)  Brousius, J. (I984) Gene 27, I5I-I60.

(24)  Brosius J., (I984), Gene, 27 I6I-I72.

(25)  Messing J., (I983) Methods in Enzymology, I0I, 20-78.

(26)    Benton W.D. and Davis R.W. (1977), Science 196, 180-182.

(27)    Japanese Patent Application No. 59-274478.

(28) Mizusawa S, and Gottesman S. (1983), Proc. Natl. Acad. Sci. USA 80, 358-362.

(29)    Hung P.P., Japanese Unexamined Patent Publication No. 56-158799 (EP-A-37687).

(30)    Laemmli, U.K. (1970) Nature 227, 680-685.

## Claims

1. A stabilized human prourokinase-like polypeptide having an amino acid sequence of the following formula :

$$\begin{array}{ccc} 1 & 135 & 157 \\ (Met) - Ser \longrightarrow X \longrightarrow Y \longrightarrow \end{array}$$

wherein Met is a methionine optionally present ; Ser is a serine at position 1 of N terminal, X is either a lysine or an amino acid other than basic amino acid at position 135, and Y is either phenylalanine or an acidic amino acid at position 157 with the proviso that where X is lysine, Y is not phenylalanine, and the horizontal lines are the amino acid sequences identical with corresponding parts of the amino acid sequence of naturally occurring human prourokinase or the derivatives thereof, which are generated by replacing or removing amino acids other than X and Y or by adding amino acids and retaining the physiological property and stability of the prourokinaselike polypeptide carrying the above-mentioned amino acids at positions X and Y.

2. A polypeptide according to Claim 1 wherein the X is a naturally occurring amino acid selected from the group consisting of alanine, asparagine, aspartic acid, glutamine, glutamic acid, phenylalanine, glycine, isoleucine, leucine, methionine, serine, threonine, valine, tryptophan, tyrosine, and proline.

3. A polypeptide according to Claim 1 wherein the X is an amide of an acidic amino acid selected from the group consisting of aspartic acid and glutamic acid.

4. A polypeptide according to Claim 1 wherein the X is a hydroxyamino acid selected from the group consisting of serine and threonine.

5. A polypeptide according to Claim 1 wherein the X is an acidic amino acid selected from the group consisting of glutamic acid and aspartic acid.

6. A polypeptide according to Claim 1 wherein the X is a branched-chain neutral amino acid selected from the group consisting of isoleucine, leucine, and valine.

7. A polypeptide according to Claim 1 wherein the Y is either aspartic acid or glutamic acid.

8. A polypeptide according to Claim 1 wherein the X is glutamine, and the Y is phenylalanine.

9. A polypeptide according to Claim 1 wherein the X is threonine, and the Y is phenylalanine.

10. A polypeptide according to Claim 1 wherein the X is glutamic acid, and the Y is phenylalanine.

11. A polypeptide according to Claim 1 wherein the X is isoleucine, and the Y is phenylalanine.

12. A polypeptide according to Claim 1 wherein the X is lysine, and the Y is aspartic acid.

13. A polypeptide according to Claim 1 wherein X is glutamic acid, and Y is aspartic acid.

14. A polypeptide according to Claim 1 wherein X is glutamine and Y is aspartic acid.

15. A DNA segment coding for the human prourokinase-like polypeptide of Claim 1.

16. A DNA segment according to Claim 15 wherein codons coding for a plurality of N-terminal amino acids are codons used in E. coli at high frequency, and the codons other than the codons coding for said plurality of N-terminal amino acids and said X and Y are identical with codons specifying the corresponding amino acids in cDNA corresponding to mRNA derived from the human kidney.

17. A DNA segment according to Claim 15 wherein the codons coding for said plurality of N-terminal amino acids are the following codons :

```
    MET SER ASN GLU LEU HIS GLN VAL PRO SER

5' ATG AGC AAC GAG CTC CAC CAG GTT CCG TCG 3'

3' TAC TCG TTG CTC GAG GTG GTC CAA GGC AGC 5'
```

18. A DNA segment according to Claim 16 wherein said codons used in E. coli at high frequency have been selected in such a way as to prevent the fold-back of the corresponding mRNA and to prevent, at th mRNA

EP 0 210 279 B1

level, the complementary association of the nucleotide sequence in the vicinity of the Shine-Dalgarno (SD) sequence of the metapyrocatechase gene and the 5′ end of the human prourokinase gene.

19. A DNA segment according to Claim 15 wherein, where X is glutamine, it is coded by codon CAA ; where X is threonine, it is coded by codon ACA ; where Y is glutamic acid, it is coded by codon GAA ; where X is isoleucine, it is coded by codon ATA ; where Y is aspartic acid, it is coded by codon GAT ; and where Y is glutamic acid, it is coded by codon GAA.

20. A plasmid containing the DNA segment of Claim 15, controlling regions for the expression of said prourokinase-like polypeptide, and a DNA sequence necessary for the replication within the E. coli cell.

21. A plasmid according to Claim 20 which contains, as the controlling regions for expression, the tac promoter/operator, the trp promoter/operator or the $P_L$ promoter/ operator and the Shine-Dalgarno (SD) sequence of the metapyrocatechase gene derived from Pseudomonas putida and which is capable of expressing the prourokinase-like polypeptide within the E. coli cell.

22. A plasmid according to Claim 21, wherein a plurality of N-terminal amino acids of said prourokinase-like polypeptides are coded by the codons used within E. coli cells at high frequency, said codons having been selected in such a way as to prevent the fold-back of the corresponding mRNA and to prevent, at the mRNA level, the complementary association of the nucleotide sequence in the vicinity of the Shine-Dalgarno (SD)- sequence of the metapyrocatechase gene and 5′ end of the human prourokinase gene.

23. A strain of E. coli into which the plasmid of Claim 20 has been introduced.

24. A process for preparing the prourokinase-like polypeptide comprising culturing the strain of E. coli of Claim 23 and recovering the prourokinase-like polypeptide from the cells.


**Patentansprüche**

1. Ein stabilisiertes menschliches Prourokinase-ähnliches Polypeptid mit einer Aminosäuresequenz der folgenden Formel :

$$(Met) - Ser \overset{1}{\rule{2cm}{0.4pt}} X \overset{135}{\rule{2cm}{0.4pt}} Y \overset{157}{\rule{2cm}{0.4pt}}$$

worin Met ein gegebenenfalls vorhandenes Methionin ist ; Ser ist ein Serin an Position 1 des N-Terminus, X ist entweder ein Lysin oder eine andere Aminosäure als eine basische Aminosäure in Position 135, und Y ist entweder Phenylalanin oder eine saure Aminosäure in Position 157 mit der Bedingung, daß wo X Lysin ist, Y nicht Phenylanin ist, und die horizontalen Linien sind die Aminosäuresequenzen, die identisch mit den entsprechenden Teilen der Aminosäuresequenz von natürlich vorkommender menschlicher Prourokinase oder den Derivaten davon sind, die erzeugt werden durch Ersetzen oder Entfernen von anderen Aminosäuren als X und Y oder durch Hinzufügen von Aminosäuren und Beibehalten der physiologischen Eigenschaft und Stabilität des Prourokinase-ähnlichen Polypeptids, das die obenerwähnten Aminosäuren in Positionen X und Y trägt.

2. Ein Polypeptid nach Anspruch 1, worin das X eine natürlich vorkommende Aminosäure ist, ausgewählt aus der Gruppe, bestehend aus Alanin, Asparagin, Asparaginsäure, Glutamin, Glutaminsäure, Phenylalanin, Glycin, Isoleucin, Leucin, Methionin, Serin, Threonin, Valin, Tryptophan, Tyrosin und Prolin.

3. Ein Polypeptid nach Anspruch 1, worin das X ein Amid einer Aminosäure, ausgewählt aus der Gruppe, bestehend aus Asparaginsäure und Glutaminsäure ist.

4. Ein Polypeptid nach Anspruch 1, worin das X eine Hydroxyaminosäure ist, ausgewählt aus der Gruppe, bestehend aus Serin und Threonin.

5. Ein Polypeptid nach Anspruch 1, worin das X eine saure Aminosäure ist, ausgewählt aus der Gruppe, bestehend aus Glutaminsäure und Asparaginsäure.

6. Ein Polypeptid nach Anspruch 1, worin das X eine verzweigtkettige neutrale Aminosäure ist, ausgewählt aus der Gruppe, bestehend aus Isoleucin, Leucin und Valin.

7. Ein Polypeptid nach Anspruch 1, worin das Y entweder Asparaginsäure oder Glutaminsäure ist.

8. Ein Polypeptid nach Anspruch 1, worin das X Glutamin ist und das Y Phenylalanin ist.

9. Ein Polypeptid nach Anspruch 1, worin das X Threorin ist und das Y Phenylalanin ist.

10. Ein Polypeptid nach Anspruch 1, worin das X Glutaminsäure ist und das Y Phenylalanin ist.

11. Ein Polypeptid nach Anspruch 1, worin das X Isoleucin ist und das Y Phenylalanin ist.

12. Ein Polypeptid nach Anspruch 1, worin das X Lysin ist und das Y Asparaginsäure ist.

29

13. Ein Polypeptid nach Anspruch 1, worin X Glutaminsäure ist und Y Asparaginsäure ist.

14. Ein Polypeptid nach Anspruch 1, worin X Glutamin ist und Y Asparaginsäure ist.

15. Ein DNA-Segment, das für das menschliche Prourokinase-ähnliche Polypeptid von Anspruch 1 codiert.

16. Ein DNA-Segment nach Anspruch 15, worin die Codons, die für eine Anzahl an N-terminalen Aminosäuren codieren, Codons sind, die in E. coli mit großer Häufigkeit verwendet werden, und die anderen Codons als die Codons, die für die Vielfalt von N-terminalen Aminosäuren und das X und Y codieren, sind identisch mit Codons, die die entsprechende Aminsäuresequenz in cDNA spezifizieren, die der mRNA entspricht, die aus menschlicher Niere stammt.

17. Ein DNA-Segment nach Anspruch 15, worin die Codons, die für die Anzahl von N-terminalen Aminosäuren codieren, die folgenden Codons sind :

```
MET SER ASN GLU LEU HIS GLN VAL PRO SER
5' ATG AGC AAC GAG CTC CAC CAG GTT CCG TCG 3'
3' TAC TCG TTG CTC GAG GTG GTC CAA GGC AGC 5'
```

18. Ein DNA-Segment nach Anspruch 16, worin die Codons, die in E. coli mit großer Häufigkeit verwendete werden, auf solche Weise ausgewählt worden sind, daß sie das Rückfalten der entsprechenden mRNA verhindern und, auf der mRNA-Ebene, die Komplementäre Assoziation der Nucleotidsequenz in der Nachbarschaft der Shine-Dalgarno (SD)-Sequenz des Metapyrocatechasegens und dem 5'-Ende des menschlichen Prourokinasegens verhindern.

19. Ein DNA-Segment nach Anspruch 15, worin , wo X Glutamin ist, wird es durch Codon CAA codiert wird; wo X Threonin ist, wird es durch Codon ACA codiert ; wo Y Glutaminsäure ist, wird es durch Codon GAA codiert; wo X Isoleucin ist, wird es durch Codon ATA codiert ; wo Y Asparaginsäure ist, wird es durch Codon GAT codiert ; und wo Y Glutaminsäure ist, wird es durch Codon GAA codiert.

20. Ein Plasmid, enthaltend das DNA-Segment von Anspruch 15, Kontrollregionen für die Expression des Prourokinase-ähnlichen Polypeptids und eine DNA-Sequenz, die nötig ist für die Replikation in der E.coli-Zelle.

21. Ein Plasmid nach Anspruch 20, das als Kontrollregionen zur Expression den tac Promotor/Operator, den trp Promotor/Operator oder den $P_L$-Promotor/Operator und die Shine-Dalgarno (SD)-Sequenz des Metapyrocatechasegens, die aus Pseudomonas putida stammt, enthält, und das in der Lage ist, das Prourokinase-ähnliche Polypeptid in der E.coli-Zelle zu exprimieren.

22. Ein Plasmid nach Anspruch 21, worin eine Anzahl von N-terminalen Aminosäuren des Prourokinase-ähnlichen Polypeptids durch Codons codiert werden, die in E. coli-Zellen mit großer Häufigkeit verwendet werden, wobei die Codons auf solche Weise ausgewählt worden sind, daß sie das Rückfalten der entsprechenden mRNA verhindern und, auf der mRNA-Ebene, die komplementäre Assoziation der Nukleotidsequenz in der Nachbarschaft der Shine-Dalgarno (SD)-Sequenz des Metapyrocatechasegens und dem 5'-Ende des menschlichen Prourokinasegens verhindern.

23. Ein E. coli-Stamm, in den das Plasmid von Anspruch 20 eingeführt worden ist.

24. Ein Verfahren zum Herstellen des Prourokinase-ähnlichen Polypeptids, umfassend Kultivieren des E.coli-Stamms von Anspruch 23 und Gewinnen des Prourokinase-ähnlichen Polypeptids von den Zellen.

## Revendications

1. Un polypeptide de type prourokinase humaine stabilisée, caractérisé par une séquence d'acides aminés représentée par la formule suivante :

```
        1      135   157
(Met) --- Ser --- X --- Y ---
```

dans laquelle Met est une méthionine éventuellement présente ; Ser est une sérine en position 1 du N terminal, X est une lysine ou un acide aminé autre qu'un acide aminé basique en position 135 et Y est une phénylalanine ou un acide aminé acide en position 157, à condition que lorsque X est une lysine, Y ne soit pas une phénylalanine, et les lignes horizontales représentent les séquences d'acides aminés identiques aux parties correspondantes de la séquence d'acides aminés d'une prourokinase humaine d'origine naturelle ou des dérivés de celle-ci, qui sont générés par remplacement ou élimination d'acides aminés autres que X et Y ou par addition d'acides aminés et conservation de la propriété physiologique et de la stabilité du polypeptide de type prourokinase portant les acides aminés mentionnés plus haut aux positions X et Y.

2. Un polypeptide suivant la revendication 1, caractérisé en ce que X est un acide aminé d'origine naturelle choisi dans le groupe comprenant l'alanine, l'asparagine, l'acide aspartique, la glutamine, l'acide glutamique, la phénylalanine, la glycine, l'isoleucine, la leucine, la méthionine, la sérine, la thréonine, la valine, le tryptophane, la tyrosine et la proline.

3. Un polypeptide suivant la revendication 1, caractérisé en ce que X est un amide d'un acide aminé acide choisi parmi l'acide aspartique et l'acide glutamique.

4. Un polypeptide suivant la revendication 1, caractérisé en ce que X est un hydroxy-aminoacide choisi parmi la sérine et la thréonine.

5. Un polypeptide suivant la revendication 1, caractérisé en ce que X est un acide aminé acide choisi parmi l'acide glutamique et l'acide aspartique.

6. Un polypeptide suivant la revendication 1, caractérisé en ce que X est un acide aminé neutre à chaîne ramifiée choisi parmi l'isoleucine, la leucine et la valine.

7. Un polypeptide suivant la revendication 1, caractérisé en ce que Y est l'acide aspartique ou l'acide glutamique.

8. Un polypeptide suivant la revendication 1, caractérisé en ce que X est la glutamine et Y est la phénylalanine.

9. Un polypeptide suivant la revendication 1, caractérisé en ce que X est la thréonine et Y est la phénylalanine.

10. Un polypeptide suivant la revendication 1, caractérisé en ce que X est l'acide glutamique et Y est la phénylalanine.

11. Un polypeptide suivant la revendication 1, caractérisé en ce que X est l'isoleucine et Y est la phénylalanine.

12. Un polypeptide suivant la revendication 1, caractérisé en ce que X est la lysine et Y est l'acide aspartique.

13. Un polypeptide suivant la revendication 1, caractérisé en ce que X est l'acide glutamique et Y est l'acide aspartique.

14. Un polypeptide suivant la revendication 1, caractérisé en ce que X est la glutamine et Y est l'acide aspartique.

15. Un segment d'ADN codant pour le polypeptide de type prourokinase humaine suivant la revendication 1.

16. Un segment d'ADN suivant la revendication 15, caractérisé en ce que les codons codant pour un grand nombre d'acides aminés à extrémité N-terminale sont des codons utilisés dans E. coli à une fréquence élevée, et que les codons autres que les codons codant pour ce grand nombre d'acides aminés à extrémité N-terminale et ces X et Y sont identiques aux codons spécifiant les acides aminés correspondants dans l'ADNc correspondant à l'ARNm dérivé du rein humain.

17. Un segment d'ADN suivant la revendication 15, caractérisé en ce que des codons codant pour ce grand nombre d'acides aminés à extrémité N terminale, sont les codons suivants :

```
      MET SER  ASN GLU LEU  HIS  GLN VAL PRO SER
   5'   ATG AGC AAC GAG CTC CAC CAG GTT CCG TCG    3'
   3'   TAC TCG TTG  CTC GAG GTG GTC CAA GGC AGC    5'
```

18. Un segment d'ADN suivant la revendication 16, caractérisé en ce que ces codons utilisés dans E. coli à une fréquence élevée ont été choisis de façon à empêcher le repliement de l'ARNm correspondant et à empêcher, au niveau de l'ARNm, l'association complémentaire de la séquence nucléotidique au voisinage de la séquence Shine-Dalgaro (SD) du gène de la métapyrocatéchase et de l'extrémité 5′ du gène de la prourokinase humaine.

19. Un segment d'ADN suivant la revendication 15, caractérisé en ce que, lorsque X est la glutamine, il est codé par le codon CAA ; lorsque X est la thréonine, il est codé par le codon ACA ; lorsque Y est l'acide glutamique, il est codé par le codon GAA ; lorsque X est l'isoleucine, il est codé par le codon ATA ; lorsque Y est l'acide aspartique, il est codé par le codon GAT ; et lorsque Y est l'acide glutamique, il est codé par le codon GAA.

20. Un plasmide, caractérisé en ce qu'il contient le segment d'ADN suivant la revendication 15, des régions de contrôle pour l'expression de ce polypeptide de type prourokinase et une séquence d'ADN nécessaire pour la réplication dans la cellule de E.coli.

21. Un plasmide suivant la revendication 20, caractérisé en ce qu'il contient en tant que régions de contrôle pour l'expression, le promoteur/opérateur tac, le promoteur/ opérateur trp ou le promoteur/opérateur $P_L$ et la séquence Shine- Dagarno (SD) du gène de la métapyrocatéchase dérivé de Pseudomonas putida et qu'il est capable d'exprimer le polypeptide de type prourokinase dans la cellule de E.coli.

22. Un plasmide suivant la revendication 21, caractérisé en ce qu'un grand nombre d'acides aminés à extrémités N-terminale de ces polypeptides de type prourikinase sont codés par les codons utilisés dans des cellules de E. coli à haute fréquence, ces codons ayant été sélectionnés de façon à empêcher le repliement de l'ARNm correspondant et à empêcher, au niveau de l'ARNm, l'association complémentaire de la séquence nucléotidique au voisinage de la séquence Shine-Dalgarno (SD) du gène de la métapyrocatéchase et de l'extrémité 5' du gène de la prourokinase humaine.

23. Une souche de E. coli dans laquelle a été introduit le plasmide suivant la revendication 20.

24. Un procédé pour préparer la polypeptide de type prourokinase, caractérisé en ce qu'il comprend la culture de la souche E. coli de la revendication 23 et la récupération du polypeptide de type prourokinase à partir des cellules.

Fig. 1

Fig.2

Fig.3

EP 0 210 279 B1

```
                                                          met arg ala leu leu ala arg leu   8
CAGCGCCGGCTCGCGCCCTCCTGCCCGCAGCCACCGAGCCGCCGTCTAGCGCCCCGACCTCGCCACC   ATG AGA GCC CTG CTG GCG CGC CTG
1                                            50


                                                  1
leu leu cys val leu val val ser asp ser lys gly SER ASN GLU LEU HIS GLN VAL PRO SER ASN CYS ASP CYS  13
CTT CTC TGC GTC CTG GTC GTG AGC GAC TCC AAA GGC AGC AAT GAA CTT CAT CAA GTT CCA TCG AAC TGT GAC TGT
           100                                          150


LEU ASN GLY GLY THR CYS VAL SER ASN LYS TYR PHE SER ASN ILE HIS TRP CYS ASN CYS PRO LYS LYS PHE GLY  38
CTA AAT GGA GGA ACA TGT GTG TCC AAC AAG TAC TTC TCC AAC ATT CAC TGG TGC AAC TGC CCA AAG AAA TTC GGA
                                       200


GLY GLN HIS CYS GLU ILE ASP LYS SER LYS THR CYS TYR GLU GLY ASN GLY HIS PHE TYR ARG GLY LYS ALA SER  63
GGG CAG CAC TGT GAA ATA GAT AAG TCA AAA ACC TGC TAT GAG GGG AAT GGT CAC TTT TAC CGA GGA AAG GCC AGC
         250                                                        300


THR ASP THR MET GLY ARG PRO CYS LEU PRO TRP ASN SER ALA THR VAL LEU GLN GLN THR TYR HIS ALA HIS ARG  88
ACT GAC ACC ATG GGC CGG CCC TGC CTG CCC TGG AAC TCT GCC ACT GTC CTT CAG CAA ACG TAC CAT GCC CAC AGA
                                          350


SER ASP ALA LEU GLN LEU GLY LEU GLY LYS HIS ASN TYR CYS ARG ASN PRO ASP ASN ARG ARG ARG PRO TRP CYS 113
TCT GAT GCT CTT CAG CTG GGC CTG GGG AAA CAT AAT TAC TGC AGG AAC CCA GAC AAC CGG AGG CGA CCC TGG TGC
         400                                                        450


TYR VAL GLN VAL GLY LEU LYS LEU PRO VAL GLN GLU CYS MET VAL HIS ASP CYS ALA ASP GLY LYS LYS PRO SER 138
TAT GTG CAG GTG GGC CTA AAG CCG CTT GTC CAA GAG TGC ATG GTG CAT GAC TGC GCA GAT GGA AAA AAG CCC TCC
                                       500


SER PRO PRO GLU GLU LEU LYS PHE GLN CYS GLY GLN LYS THR LEU ARG PRO ARG PHE LYS ILE ILE GLY GLY GLU 163
TCT CCT CCA GAA GAA TTA AAA TTT CAG TGT GGC CAA AAG ACT CTG AGG CCC CGC TTT AAG ATT ATT GGG GGA GAA
         550                                                        600
```

Fig.4-1

PHE THR THR ILE GLU ASN GLN PRO TRP PHE ALA ALA ILE TYR ARG ARG HIS ARG GLY GLY SER VAL THR TYR VAL 188
TTC ACC ACC ATC GAG AAC CAG CCC TGG TTT GCG GCC ATC TAC AGG AGG CAC CGG GGG GGC TCT GTC ACC TAC GTG
                                                      ·650

CYS GLY GLY SER LEU ILE SER PRO CYS TRP VAL ILE SER ALA THR HIS CYS PHE ILE ASP TYR PRO LYS LYS GLU 213
TGT GGA GGC AGC CTC ATC AGC CCT TGC TGG GTG ATC AGC GCC ACA CAC TGC TTC ATT GAT TAC CCA AAG AAG GAG
        700                                                                          750

ASP TYR ILE VAL TYR LEU GLY ARG SER ARG LEU ASN SER ASN THR GLN GLY GLU MET LYS PHE GLU VAL GLU ASN 238
GAC TAC ATC GTC TAC CTG GGT CGC TCA AGG CTT AAC TCC AAC ACG CAA GGG GAG ATG AAG TTT GAG GTG GAA AAC
                                              800

LEU ILE LEU HIS LYS ASP TYR SER ALA ASP THR LEU ALA HIS HIS ASN ASP ILE ALA LEU LEU LYS ILE ARG SER 263
CTC ATC CTA CAC AAG GAC TAC AGC GCT GAC ACG CTT GCT CAC CAC AAT GAC ATT GCC TTG CTG AAG ATC CGT TCC
        850                                                                    900

LYS GLU GLY ARG CYS ALA GLN PRO SER ARG THR ILE GLN THR ILE CYS LEU PRO SER MET TYR ASN ASP PRO GLN 288
AAG GAG GGC AGG TGT GCG CAG CCA TCC CGG ACT ATA CAG ACC ATC .TGC CTG CCC TCG ATG TAT AAC GAT CCC CAG
                                              950

PHE GLY THR SER CYS GLN ILE THR GLY PHE GLY LYS GLU ASN SER THR ASP TYR LEU TYR PRO GLU GLN LEU LYS 313
TTT GGC ACA AGC TGT GAG ATC ACT GGC TTT GGA AAA GAG AAT TCT ACC GAC TAT CTC TAT CCG GAG CAG CTG AAA
        1000                                                                       1050

MET THR VAL VAL LYS LEU ILE SER HIS ARG GLU CYS GLN GLN PRO HIS TYR TYR GLY SER GLU VAL THR THR LYS 338
ATG ACT GTT GTG AAG CTG ATT TCC CAC CGG GAG TGT CAG CAG CCC CAC TAC TAC GGC TCT GAA GTC ACC ACC AAA
                                              1100

MET LEU CYS ALA ALA ASP PRO GLN TRP LYS THR ASP SER CYS GLN GLY ASP SER GLY GLY PRO LEU VAL CYS SER 363
ATG CTG TGT GCT GCT GAC CCA CAG TGG AAA ACA GAT TCC TGC CAG GGA GAC TCA GGG GGA CCC CTC GTC TGT TCC
        1150                                                                       1200

Fig.4-2

EP 0 210 279 B1

EP 0 210 279 B1

LEU GLN GLY ARG MET THR LEU THR GLY ILE VAL SER TRP GLY ARG GLY CYS ALA LEU LYS ASP LYS PRO GLY VAL 388
CTC CAA GGC CGC ATG ACT TTG ACT GGA ATT GTG AGC TGG GGC CGT GGA TGT GCC CTG AAG GAC AAG CCA GGC GTC
                                        1250

TYR THR ARG VAL SER HIS PHE LEU PRO TRP ILE ARG SER HIS THR LYS GLU GLU ASN GLY LEU ALA LEU XXX     411
TAC ACG AGA GTC TCA CAC TTC TTA CCC TGG ATC CGC AGT CAC ACC AAG GAA GAG AAT GGC CTG GCC CTC TGA GGG
         1300                                                                    1350

TCCCCAGGGAGGAAACGGGCACCACCCGCTTTCTTGCTGGTTGCTATTTTGCAGTAGAGTCATCTCCATCAGCTGTAAGAAGAGACTGGGAAGATAGGCT
                      1400                                                        1450

CTGCACAGATGGATTTGCCTGTGCCCACCACCAGGGCGAACGACAATAGCTTTACCCTCAGGCATAGGCCTGGGTGCTGGCTGCCCAGACCCCTCTGGCC
                  1500                                                            1550

AGGATGGAGGGGTGGTCCTGACTCAACATGTTACTGACCAGCAACTTGTCTTTTTCTGGACTGAAGCCTGCAGGAGTTAAAAAGGGCAGGGCATCTCCT
                 1600                                                             1650

GTGCATGGGCTCGAAGGGAGAGCCAGCTCCCCCGAGCGGTGGGCATTTGTGAGGCCCATGGTTGAGAAATGAATAATTTCCCAATTAGGAAGTGTAAGC
                 1700                                                             1750

AGCTGAGGTCTCTTGAGGGAGCTTAGCCAATGTGGGAGCAGCGGTTTGGGGAGCAGAGACACTAACGACTTCAGGGCAGGGCTCTGATATTCCATGAAT
                 1800                                                             1850

GTATCAGGAAATATATATGTGTGTGTATGTTTGCACACTTGTGTGTGTGGGCTGTGAGTGTAAGTGTGAGAAAGAGCTGGTGTCTGATTGTTAAGTCTAAA
                 1900                                                             1950

TATTTCCTTAAACTGTGTGGACTGTGATGCCACACAGAGTGGTCTTTCTGGAGAGGTTATAGGTCACTCCTGGGGCTCTTGGGTCCCCCACGTGACAG
                 2000                                                             2050

TGCCTGGGAATGTATTATTCTGCAGCATGACCTGTGACCAGCACTGTCTCAGTTTCACTTTCACATAGATGTCCCTTTCTTGGCCAGTTATCCCTTCCT
                2100                                                              2150

TTTAGCCTAGTTCATCCAATCCTCACTGGGTGGGGTGAGGACCACTCCTGTACACTGAATATTTATATTTCACTATTTTTTATTTATATTTTTTGTAATTT
                2200                                                              2250

TAAATAAAAGTGATCAATAAAATGTGATTTTTCTGATGAAAAA
                2294

                                        Fig.4-3

Fig. 5

EP 0 210 279 B1

Fig.6

Fig.7

Primer

```
                         Gly  Thr  Lys  Pro  Ser  Ser
                         GGA  ACA  AAG  CCC  TCC  TCT  3'          Primer      (2)
                    Asp  Gly  Gln  Lys  Pro
                5' GAT GGA CAA AAG CCC 3'                          Primer      (1)
            3'---- CTA CCT TTT TTC GGG AGG AGA ----5'              Single-stranded Template DNA
                    Asp  Gly  Lys  Lys  Pro  Ser  Ser
```

DNA

Annealing 60°C, 20 min., 20°C, 20 min.
DNA polymerase Klenow fragment + dNTPs
T4DNA ligase

S1 nuclease digestion
Transformation into E. coli JM103

Screening by plaque
hybridization

Plaque

Fig. 8

Fig.9

Fig.10

Polylinker

HindⅢ  PstI  BamHI  EcoRI
       SalI  SmaI

rrBT1T2  Ptac  BamHI

Amp^r     pKK223-3     Tet^s  SalI
          4.6Kb

EcoRI ClaI Ptrp
Pvu Ⅱ

pSTN16
Amp^r  2.6Kb

PvuⅡ

Cla I-PvuⅡ                              ECO RI-PvuⅡ
300bp fragment isolation               2.6Kb fragment isolation
Repair by Klenow                       Repair by Klenow
                                       BAP

5'CGAT Ptrp CAG        T4DNA ligase    5'CTG  Amp^r  GAATT
 GCTA       GTC                         GAC          CTTAA

                      Transformation into
                      E.coli HB 101

                      Plasmid check by Eco RI-Pvu II digestion

Polylinker

HindⅢ  PstI  BamHI  EcoRI
       SalI  SmaI

rrBT1T2  Ptrp
              PvuⅡ

pKKtrp
Amp^r  2.9Kb

Fig.11

Fig.12

A        B

1 2 3 4 5    1 2 3 4 5

High molecular urokinase
(54K)
Low molecular urokinase (33K)

1-hour Incubation    2-hour Incubation

Fig.13

1  2  3  4

High molecular urokinase (54K)

Low molecular urokinase (33K)

Fig.14

Fig.15

Fig.16

Fig.17